# EUROPEAN PATENT APPLICATION

(11) **EP 4 659 670 A1**
(43) Date of publication of application: **10.12.2025**
(21) Application number: 25178238.9
(22) Date of filing: 22.05.2025
(51) Int. Cl.: A61B 5/15, A61B 5/151

(54) **DERMAL PATCH FOR COLLECTING A PHYSIOLOGICAL SAMPLE**

(30) Priority: 24.05.2024 US 202418674275
(71) Applicant: Satio, Inc., Boston, Massachusetts 02210 (US)
(72) Inventor: NAWANA, Namal, Weston, MA 02493 (US); MONIZ, Mike, Boston, MA 02210 (US); AL-SHAMSIE, Ziad Tarik, San Diego, CA 92103 (US)
(74) Representative: Grünecker Patent- und Rechtsanwälte PartG mbB

(57) **Abstract**

A system for collecting a physiological sample from a subject includes a lancet configured to draw a physiological sample and a cartridge configured to be affixed to the subject's skin. The cartridge includes a physiological sample well, a sloped physiological sample channel in open communication with the physiological sample well and a sample collection pad in in open communication with the sloped physiological channel and configured to absorb the drawn physiological sample. The physiological sample well is configured to retain the drawn physiological sample and the sloped physiological channel is configured to carry the drawn physiological sample from the physiological sample to the sample collection pad.

## Description

### RELATED APPLICATIONS

The present application claims priority to U.S. Application No. 17/719,881 filed on April 13, 2022 (4378-0010US01) which claims priority to U.S. Provisional Application No. 63/174,956 filed on April 14, 2021 (4378-0010PV01 ), U.S. Application No. 17/412,205 filed on August 25, 2021 (4378-0013US01) which claims priority to U.S. Provisional Application No. 63/190,700 filed on May 19, 2021 (4378-0013PV01) and U.S. Provisional Application No. 63/174,956 filed on April 14, 2021 (4378-0010PV01), U.S. Application No. 17/903,802 filed on September 6, 2022 (4378-0014US02) which claims priority to U.S. Provisional Application No. 63/190,700 filed on May 19, 2021 (4378-0013PV01), U.S. Provisional Application No. 63/174,956 filed on April 14, 2021 (4378-0010PV01) and U.S. Provisional Application No. 17/412,213 filed on August 25, 2021 (4378-0014US01), U.S. Application No. 18/090,026 filed on December 28, 2022 (4378-0016US03) which claims priority to U.S. Application No. 17/500,873 filed on October 13, 2021 (4378-0015US01), U.S. Application No. 17/903,802 filed on September 6, 2022 (4378-0014US02), U.S. Application No. 17/971,142 filed on October 21, 2022 (4378-0018US01), U.S. Application No. 17/991,284 filed on November 21, 2022 (4378-0019US01) and U.S. Application No. 17/994,454 filed on November 28, 2022 (4378-0016US02), U.S. Application No. 17/971,142 filed on October 21, 2022 (4378-0018US01) which claims priority to U.S. Application No. 17/521,466 filed on November 8, 2021 (4378-0016US01), U.S. Application No. 18/090,063 filed on December 28, 2022 (4378-0020US01) which claims priority to U.S. Application No. 17/500,873 filed on October 13, 2021 (4378-0015US01), U.S. Application No. 17/903,802 filed on September 6, 2022 (4378-0014US02), U.S. Application No. 17/971,142 filed on October 21, 2022 (4378-0018US01), U.S. Application No. 17/991,284 filed on November 21, 2022 (4378-0019US01) and U.S. Application No. 17/994,454 filed on November 28, 2022 (4378-0016US02), U.S. Application No. 18/597,513 filed on March 6, 2024 (4378-0022US01) which claims priority to U.S. Provisional Application No. 63/174,956 filed on April 14, 2021 (4378-0010PV01), U.S. Provisional No. 63/190,700 filed on May 19, 2021 (4378-0013PV01), U.S. Application No. 17/412,205 filed on August 25, 2021 (4378-0013US01), U.S. Application No. 17/719,881 filed on April 13, 2022 (4378-0010US01), U.S. Application No. 17/747,544 filed on May 18, 2022 (4378-0013US02), U.S. Application No. 17/903,802 filed on September 6, 2022 (4378-0014US02), U.S. Application No. 17/971,142 filed on October 21, 2022 (4378-0018US01), 17/991,284 filed on November 21, 2022 (4378-0019US01), U.S. Application No. 18/090,026 filed on December 28, 2022 (4378-0016US03) and U.S. Application No. 18/090,063 filed on December 28, 2022 (4378-0020US01), U.S. Application No. 18/596,098 filed on March 5, 2024 (4378-0024US01) which claims priority to U.S. Application No. 63/174,956 filed on April 14, 2021 (4378-0010PV01), U.S. Provisional Application No. 63/190,700 filed May 19, 2021 (4378-0013PV01), U.S. Application No. 17/412,205 filed on August 25, 2021 (4378-0013US01), U.S. Application No. 17/412,213 filed on August 25, 2021 (4378-0014US01), U.S. Application No. 17/500,873 filed on October 13, 2021 (4378-0015US01), U.S. Application No. 17/521,466 filed on November 8, 2021 (4378-0016US01), U.S. Application No. 17/719,881 filed on April 13, 2022 (4378-0010US01), U.S. Application No. 17/747,544 filed May 18, 2022 (4378-0013US02), U.S. Application No. 17/903,802 filed on September 6, 2022 (4378-0014US02), U.S. Application No. 17/971,142 filed on October 21, 2022 (4378-0018US01), U.S. Application No. 17/991,284 filed on November 21, 2022 (4378-0019US01), U.S. Application No. 17/994,454 filed November 28, 2022 (4378-0016US02), U.S. Application No. 18/090,026 filed on December 28, 2022 (4378-0016US03) and U.S. Application No. 18/090,063 filed on December 28, 2022 (4378-0020US01).

The present application is also related to utility application entitled Dermal Patch for Collecting a Physiological Sample which is filed concurrently herewith and is hereby incorporated by reference in its entirety.

### TECHNICAL FIELD

The present teachings are generally directed to a lancet for use with a cartridge of a dermal patch system (also referred to as "dermal patches" herein).

### BACKGROUND

Several diagnostic tests may be completed by analyzing a physiological sample from a subject. In order to obtain the physiological sample, a subject may have to travel to a diagnostic lab, wherein a medical professional obtains the sample with a standard syringe. This process may be time consuming and burdensome to the subject. At home solutions, may allow a user to collect the sample with a needle that, after use, may not be properly disposed of which may result in accidental injury.

### SUMMARY

Aspects of the present disclosure address the above-referenced problems and/or others and satisfy an unmet need for a safe lancet that can draw a physiological sample from a subject.

In one aspect, a system for collecting a physiological sample from a subject includes a lancet configured to draw a physiological sample, and a cartridge configured to be affixed to the subject's skin. The cartridge includes a physiological sample well, a sloped physiological sample channel in open communication with the physiological sample well, and a sample collection pad in open communication with the sloped physiological channel and configured to absorb the drawn physiological sample. The physiological sample well is configured to retain the drawn physiological sample and the sloped physiological channel is configured to carry the drawn physiological sample from the physiological sample well to the sample collection pad. In some embodiments the system can include a desiccant disposed within the cartridge, but a desiccant is not necessary. In some embodiments, the system further includes a hydrophobic foam disposed within the cartridge and configured to prevent the drawn physiological sample from escaping the sample collection pad. In some embodiments, the sample collection pad can comprise sections with tabs therebetween. In some embodiments, the cartridge can comprise a bottom layer, an adhesive layer attached to the bottom layer, a top layer, a sample well opening formed in the adhesive layer, a needle aperture formed in the top layer, wherein the needle aperture is smaller than the sample well opening. The desiccant can be formed in a space between the top layer and the bottom layer of the cartridge. The adhesive layer can be formed of a pressure sensitive adhesive, and can include at least one cutout vent hole configured to allow the physiological sample on the sample collection pad to dry faster. The top layer can include a cutout configured to hold an integrated overflow filter.

In some embodiments, the sample collection pad is a first sample collection pad and the cartridge includes a second collection pad. The physiological sample channel is configured to carry the drawn physiological sample to the first and second collection pad. In some embodiments, the lancet includes one or more needles configured to move between an undeployed position and a deployed position, wherein the one or more needles are disposed within the lancet when in the undeployed position and extends out of the lancet when in the deployed position to draw the physiological sample. In some embodiments, the cartridge includes a lancet receiving element. The lancet is configured to move the one or more needles from the undeployed position to the deployed position when the lancet engages with the lancet receiving element, or when a configuration of the lancet engages with a matching configuration of the cartridge. In some embodiments, the lancet is configured to automatically retract one or more needles into a housing of the lancet. In some embodiments, the system includes a vacuum pin disposed within the cartridge and configured to move between a deployed position and an undeployed position. The vacuum pin is configured to create a vacuum within the cartridge when moved from the undeployed position to the deployed position and the vacuum draws the drawn physiological sample from the physiological sample well to the sample collection pad. In some embodiments, the cartridge includes a first layer of material, and a second layer of material. The first and second layer of material define the physiological sample channel.

In another aspect, a system for collecting a physiological sample from a subject includes a lancet configured to draw a physiological sample and a cartridge configured to receive and store the physiological sample. The cartridge includes a cover with a first portion and a second portion, wherein the first portion is removable, a first quick response code disposed on the first portion of the cover, and a second quick response code disposed on the second portion. The first and second quick response codes are associated with the cartridge. In some embodiments, the cartridge further includes a sample collection pad. The cover seals the sample collection pad within the cartridge. In some embodiments, the cover includes a transparent portion that allows a user to view the sample collection pad. In some embodiments, the cover includes a third portion that covers the sample collection pad and includes the transparent portion, wherein the third portion is removable.

In some embodiments, the lancet includes one or more needles that are moveable between an undeployed position and a deployed position. The one or more needles are configured to draw the physiological sample when in the deployed position. The cartridge includes a lancet receiving element configured to cause the lancet to move the one or more needles to the deployed position. The first portion covers the lancet receiving element. In some embodiments, the first portion includes a pull tab which allows a user to remove the first portion. In some embodiments, the cartridge includes a moveable vacuum pin configured to create a vacuum within the cartridge when moved. The vacuum draws the physiological sample to the collection pad.

In another aspect, a method for drawing a physiological sample includes affixing a cartridge of a dermal patch system to the skin of a subject, engaging a lancet with the cartridge to draw a physiological sample, removing the cartridge from the skin of the subject, and determining if chain of custody of the cartridge has been preserved by scanning a quick response code of the cartridge. In some embodiments, the lancet includes one or more needles that are configured to move from an undeployed position to a deployed position upon engagement with the cartridge. In some embodiments, the lancet is configured to automatically retract the one or more needles. In some embodiments, the method further includes pulling a vacuum pin of the cartridge to draw the physiological sample to a collection pad of the cartridge.

In another embodiment, the one or more needles can be coated with silicone. In another embodiment, the cartridge can include alignment ridges to align the bottom layer of the cartridge with the top layer of the cartridge. In another embodiment, the collection pad can be made from one piece having tabs formed at a middle portion thereof, thereby forming a first section and a second section that are configured to be separated from each other when the first and second section are pulled apart. The first section and the second section each can be approximately 8mm by 8mm, or approximately 16mm by 15mm.

In another embodiment, the sample collection pad can be configured to separate a plasma sample from the physiological sample. In another embodiment, the sample collection pad can comprise a blood separation section and a conjugate release section. The blood separation section can comprise a separator selected from a group consisting of an LF1 separator, an MF1 separator, a VF2 vertical separator, a Fusion 5 separator, and GF/DVA bound glass separator. The conjugate release section can comprise a conjugate release element selected from a group consisting of a Std14, Std 17 and Fusion 5. In another embodiment, the sample collection pad can comprise a plasma separation stack up. The plasma separation stack up can comprise a membrane configured to remove white blood cells, a membrane configured to remove red blood cells and a layer configured to provide a wicking source and plasma storage. In another embodiment, the plasma separation stack up comprises top clear laminate layer configured to allow viewing of the plasma sample, a polyester membrane located below the top clear laminate layer and configured to remove white blood cells, a first double-sided adhesive located below the polyester membrane and configured to adhere to the polyester membrane, an asymmetric polysulfone layer located below the first double-sided adhesive and configured to remove red blood cells (RBC's), a second double-sided adhesive located below the asymmetric polysulfone layer and configured to adhere to the asymmetric polysulfone layer, a wax-patterned cellulose layer located below the second double-sided adhesive and configured to provide a wicking source and plasma storage, a third double-sided adhesive located below the wax-patterned cellulose layer and configured to adhere to the wax-patterned cellulose layer, and a clear laminate bottom layer located below the third double-sided adhesive. Another embodiment comprises an overflow area in the vicinity of the pad configured to limit the saturation of blood on the pad in order to keep the collected blood volume within a targeted range of blood volume.

### BRIEF DESCRIPTION OF THE DRAWINGS

Aspects of the present disclosure may take form in various components and arrangements of components, and in various steps and arrangements of steps. The drawings are only for illustration purpose of preferred embodiments of the present disclosure and are not to be considered as limiting.

Features of embodiments of the present disclosure will be more readily understood from the following detailed description take in conjunction with the accompanying drawings in which:
**Fig.** 1 depicts a dermal patch system in accordance with an exemplary embodiment of the present disclosure;
**Fig. 2** depicts a lancet with three needles in accordance with an exemplary embodiment of the present disclosure;
**Fig. 3** is a cross sectional view of the lancet, wherein the lancet is in an undeployed position in accordance with an exemplary embodiment of the present disclosure;
**Fig. 4** is a cross sectional view of a housing of the lancet in accordance with an exemplary embodiment of the present disclosure;
**Fig. 5** shows an inner cap of the housing in accordance with an exemplary embodiment of the present disclosure;
**Fig. 6** depicts a side of an inner sleeve of the lancet in accordance with an exemplary embodiment of the present disclosure;
**Fig. 7** depicts another side of the inner sleeve of the lancet in accordance with an exemplary embodiment of the present disclosure;
**Fig. 8** is a cross sectional view of the inner sleeve of the lancet in accordance with an exemplary embodiment of the present disclosure;
**Fig. 9** depicts a side of a needle platform of the lancet in accordance with an exemplary embodiment of the present disclosure;
**Fig. 10** depicts another side of the needle platform in accordance with an exemplary embodiment of the present disclosure;
**Fig. 11** is a cross sectional view of a spring sleeve of the lancet in accordance with an exemplary embodiment of the present disclosure;
**Fig. 12** depicts a cartridge of the dermal patch system in accordance with an exemplary embodiment of the present disclosure;
**Fig. 13** is an exploded view of the cartridge in accordance with an exemplary embodiment of the present disclosure;
**Fig. 14** depicts an adhesive layer of the cartridge in accordance with an exemplary embodiment of the present disclosure;
**Fig. 15** depicts a bottom layer of the cartridge in accordance with an exemplary embodiment of the present disclosure;
**Fig. 16** depicts a collection pad support of the bottom layer cartridge in accordance with an exemplary embodiment of the present disclosure;
**Fig. 17** depicts a desiccant of the cartridge in accordance with an exemplary embodiment of the present disclosure;
**Fig. 18** depicts a top layer of the cartridge in accordance with an exemplary embodiment of the present disclosure;
**Figs. 19A - 19C** show another depiction of the top layer of the cartridge in accordance with an exemplary embodiment of the present disclosure;
**Fig. 20** is a bottom view of the top layer of the cartridge in accordance with an exemplary embodiment of the present disclosure;
**Fig. 21** depicts a lancet receiving element of the top layer in accordance with an exemplary embodiment of the present disclosure;
**Fig. 22** depicts the cartridge of the dermal patch system without a cover and collection pads in accordance with an exemplary embodiment of the present disclosure;
**Fig. 23** is a cross sectional view of the cartridge in accordance with an exemplary embodiment of the present disclosure;
**Fig. 24** depicts a vacuum pin of the cartridge in accordance with an exemplary embodiment of the present disclosure;
**Fig. 25** depicts a cover of the dermal patch system in accordance with an exemplary embodiment of the present disclosure;
**Fig. 26** depicts the cartridge of the dermal patch system with a portion of the cover removed in accordance with an exemplary embodiment of the present disclosure;
**Fig. 27** is a cross sectional view of the lancet engaging with the cartridge in accordance with an exemplary embodiment of the present disclosure;
**Fig. 28** is a cross sectional of the lancet transitioning to a deployed position in accordance with an exemplary embodiment of the present disclosure;
**Fig. 29** is a cross sectional of the lancet transitioning to a deployed position in accordance with an exemplary embodiment of the present disclosure;

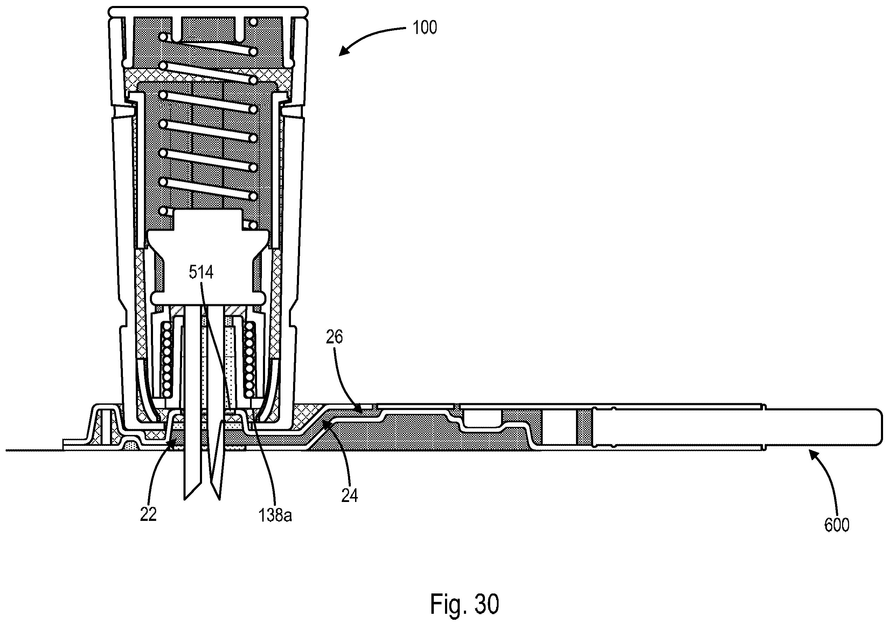

**Fig. 30** is a cross sectional view of the lancet engaging with the cartridge, wherein the lancet is in a deployed position in accordance with an exemplary embodiment of the present disclosure;
**Fig. 31** is a cross sectional view of the lancet in a deployed position in accordance with an exemplary embodiment of the present disclosure;
**Fig. 32** is a cross sectional view of the lancet engaging with the cartridge, wherein the lancet is in a retracted position in accordance with an exemplary embodiment of the present disclosure;

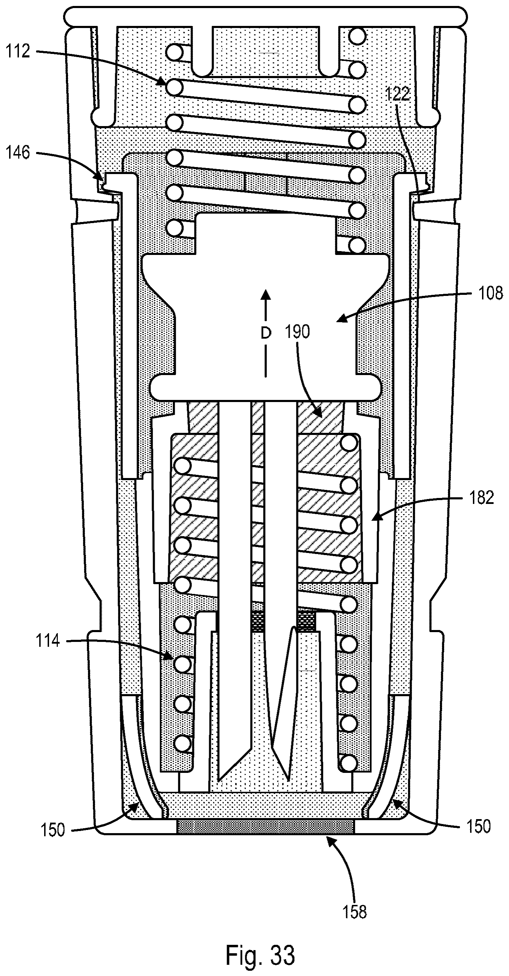

**Fig. 33** is a cross sectional view of the lancet, wherein the lancet is in a retracted position in accordance with an exemplary embodiment of the present disclosure;
**Fig. 34** is a cross sectional view of the lancet engaging with the cartridge, wherein the lancet is in a retracted position in accordance with an exemplary embodiment of the present disclosure;
**Fig. 35** depicts the cartridge of the dermal patch system including two quick response (QR) codes in accordance with an exemplary embodiment of the present disclosure;
**Fig. 36** depicts a computer system scanning the quick response codes of the cartridge in accordance with an exemplary embodiment of the present disclosure;
**Fig. 37** depicts the computer system connected to a quick response code database in accordance with an exemplary embodiment of the present disclosure;
**Fig. 38** depicts a first computer system scanning a first quick response code of the cartridge and a second computer system scanning a second quick response code of the cartridge in accordance with an exemplary embodiment of the present disclosure;
**Fig. 39** is a flow chart of a method for obtaining a physiological sample in accordance with an exemplary embodiment of the present disclosure;
**Fig. 40** schematically depicts a computer system in accordance with an exemplary embodiment of the present disclosure; and
**Fig. 41** schematically depicts a cloud computing environment in accordance with an exemplary embodiment;
**Fig. 42** shows example geometry and needle dimensions of an illustrative embodiment;
**Fig. 43** depicts a bottom layer of the cartridge in accordance with another embodiment of the present disclosure;
**Fig. 44** depicts a top layer of the cartridge in accordance with another embodiment of the present disclosure;
**Fig. 45** depicts a collection pad in accordance with another embodiment of the present disclosure.
**Fig. 46** shows a collection pad that can collect a larger physiological sample.
**Fig. 47** shows an example plasma filter.
**Fig. 48** shows a sample collection pad that can be used to collect plasma.

### DETAILED DESCRIPTION

The present disclosure generally relates to a dermal patch system (which may also be referred to as a "dermal patch") that may be utilized to collect and optionally store a physiological sample.

In various embodiments, a dermal patch system may be used to collect a physiological sample and the collected sample may then be stored on a sample collection pad of the dermal patch system. Dermal patch systems disclosed herein may allow for the collection and analysis of a physiological sample in a variety of environments (e.g., at home, in the field, in a medical facility, etc.).

The term "about," as used herein, denotes a deviation of at most 10% relative to a numerical value. For example, about 100 µm means in the range of 90 µm-110 µm.

The term "substantially," as used herein, refers to a deviation, if any, of at most 10% from a complete state and/or condition.

The term "majority," as used herein, refers to more than 50% of an object or substance.

The term "subject" as used herein refers to a human subject or an animal subject (i.e., chicken, pig, cattle, dog, cat, etc.).

The term "transparent," as used herein, indicates that light can substantially pass through an object (e.g., a window) to allow visualization of a material disposed behind the object. For example, in some embodiments, a transparent object allows the passage of at least 70%, or at least 80%, or at least 90% of visible light therethrough.

The term "needle" as used herein, refers to a component with a pointed tip that is configured to pierce an outer surface of an element (e.g., skin of a subject) to provide a passageway through which a physiological fluid can be extracted.

The term "vacuum," as used herein, refers to a pressure less than atmospheric pressure and more particularly to a pressure that can facilitate the movement of a fluid (e.g., a physiological sample) within a dermal patch system according to various embodiments.

The present disclosure generally relates to a device, which is herein also referred to as a dermal patch or a dermal patch system, for collecting a physiological sample (e.g., bodily fluids such as blood, interstitial fluids, etc.) from a subject. In some embodiments discussed below, such a dermal patch system can include a cartridge that can be affixed to a subject's skin (e.g., via an adhesive layer) and a separate lancet that can be engaged with the cartridge to puncture the skin, thereby providing a passageway for extracting the physiological sample. As discussed in more detail below, the lancet can include a housing in which at least one needle that is configured for puncturing the skin is disposed. The lancet can be transitioned between at least three states. In a first state (which may be referred to as an undeployed or a locked state) the lancet retains the needles within the housing when the lancet is not engaged with the cartridge. In a second state (which may be referred to as a deployed, an unlocked state, or a released state) the lancet allows the needles to puncture the skin in response to engagement of the lancet with the cartridge. In a third state (which may be referred to as a retracted state) the lancet automatically moves the needles from the deployed position back into the lancet housing. In other words, the engagement of the lancet with the cartridge automatically transitions the lancet from the locked state to the unlocked state, and to the retracted state. In some embodiments, the lancet includes an inner sleeve with a plurality of deformable tabs and a spring sleeve. The spring sleeve retains a lower spring in a compressed state and the deformable tabs contact the spring sleeve. This retains the spring sleeve in a first position. In the first position, the spring sleeve compresses the lower spring and prevents the lower spring from expanding.

When the lancet is engaged with the cartridge, the inner sleeve moves from a first position to a second position. In the first position, the inner sleeve retains a needle platform that supports the needles in an undeployed position. The lancet also includes an upper spring that is coupled to the needle platform. When the needle platform is in the undeployed position, the needle platform compresses the upper spring and prevents the upper spring from expanding. In the second position, the inner sleeve moves vertically upward which releases the needle platform thereby allowing the needle platform to move to a deployed position. When released, the upper spring is allowed to expand which provides a force to the needle platform and the needles extend beyond the housing of the lancet. Furthermore, this force causes the needles (which are coupled to the needle platform) to travel with sufficient velocity to puncture the skin of a subject. As the needle platform travels to the deployed position, the needle platform contacts the deformable tabs and causes the tabs to break or deflect. When broken (or deflected) the tabs no longer contact the spring sleeve which allows the lower spring to expand and move the spring sleeve vertically upward from a first position to a second position which moves the needle spring vertically upward such that the needles are retracted into the housing of the lancet.

In this manner, the lancet remains safe before it is engaged with the cartridge as the lancet is not capable of deploying the needles when the lancet is not engaged with the cartridge. Furthermore, in this manner, the lancet remains safe after drawing a physiological sample as the needles automatically retract back into the lancet's housing after being deployed.

After the physiological sample is drawn, a user of the dermal patch system pulls a vacuum pin of the dermal patch system which creates a vacuum within the cartridge. This vacuum draws the physiological sample to a collection pad of the cartridge. The collection pad retains and stores the physiological sample.

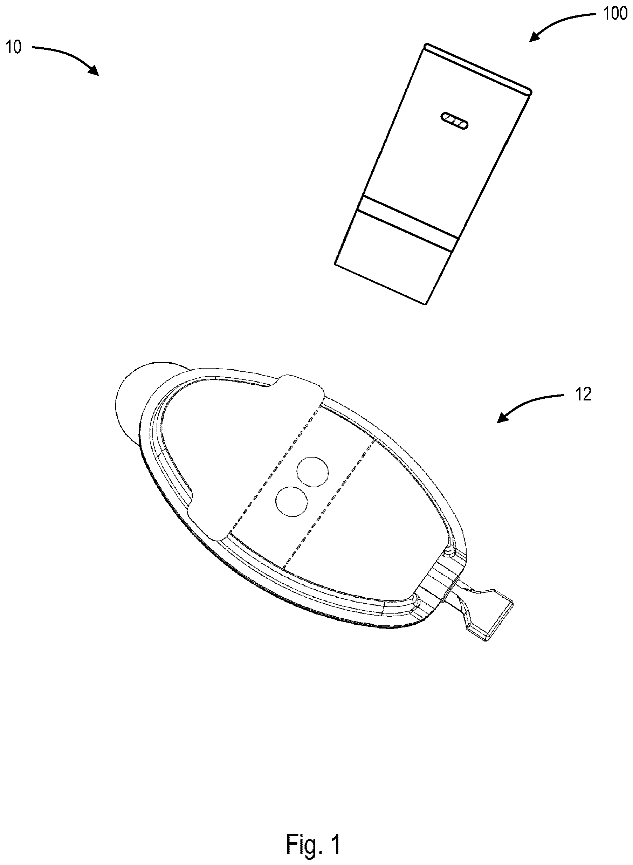

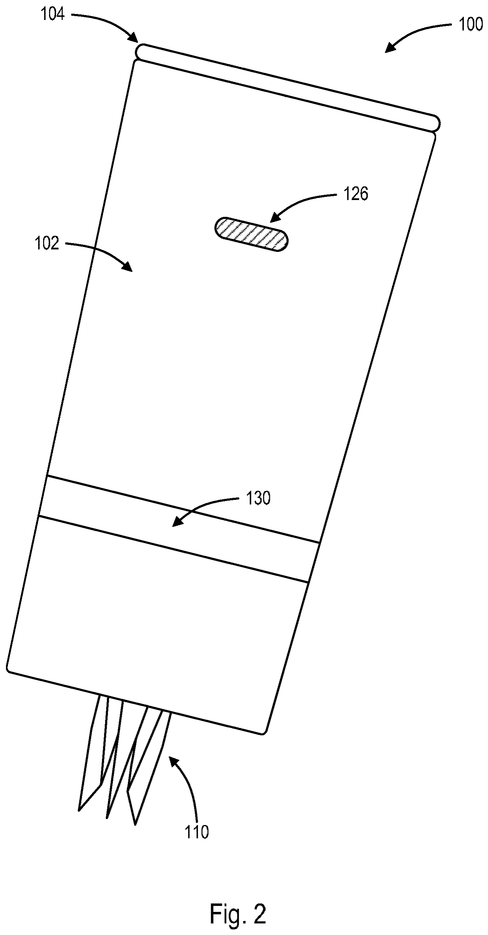

Referring now to **Figs. 1** **and** **2****,** a dermal patch system **10** is shown in accordance with an exemplary embodiment. In this embodiment, the dermal patch system **10** includes a cartridge **12** that can be affixed to a subject's skin and a lancet **100.**

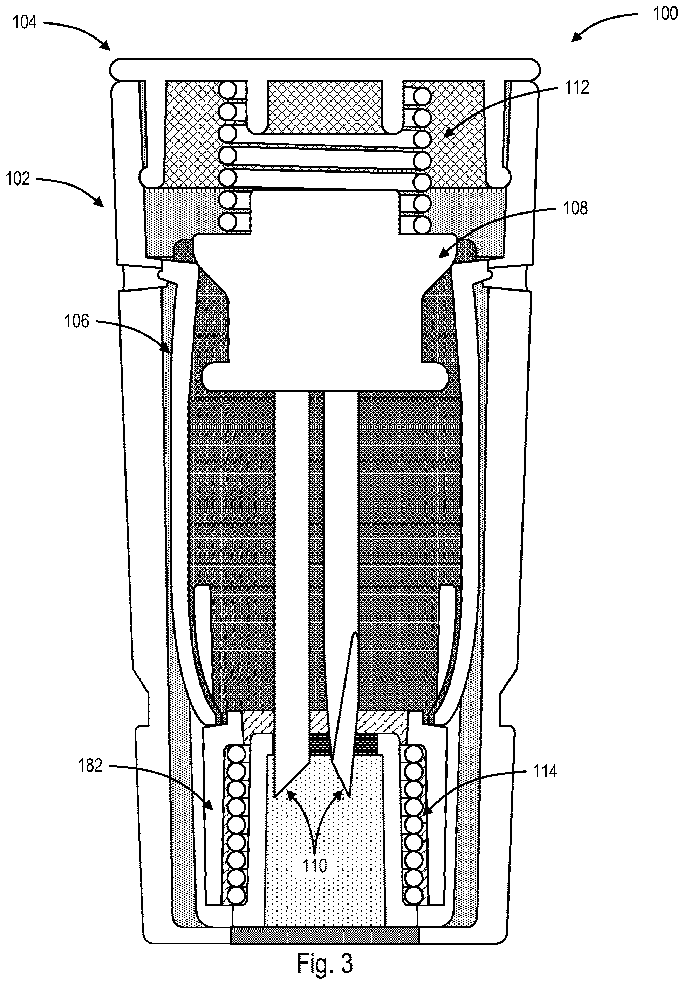

With reference to **Fig. 3** a cross sectional view of the lancet **100** is shown in accordance with an exemplary embodiment. The lancet **100** includes a housing **102** and a cap **104** that is coupled to the housing **102.** The housing **102** and the cap **104** define an inner volume of the lancet **100** in which various components of the lancet **100** are disposed. The lancet **100** further includes an inner sleeve **106** and a needle platform **108** that are disposed within the inner volume of the lancet **100.** The needle platform **108** supports a plurality of needles **110,** specifically three needles **110.** While needle platform **108** is depicted as supporting three needles **110 (****Fig. 2****),** in other embodiments, the needle platform **108** can support more or less needles **110** (e.g., 1, 2, 4, 5, needles etc.). The lancet **100** further includes an injection spring (also referred to as an upper spring) **112** and a retraction spring (also referred to as a lower spring) **114** that move the needle platform **108.**

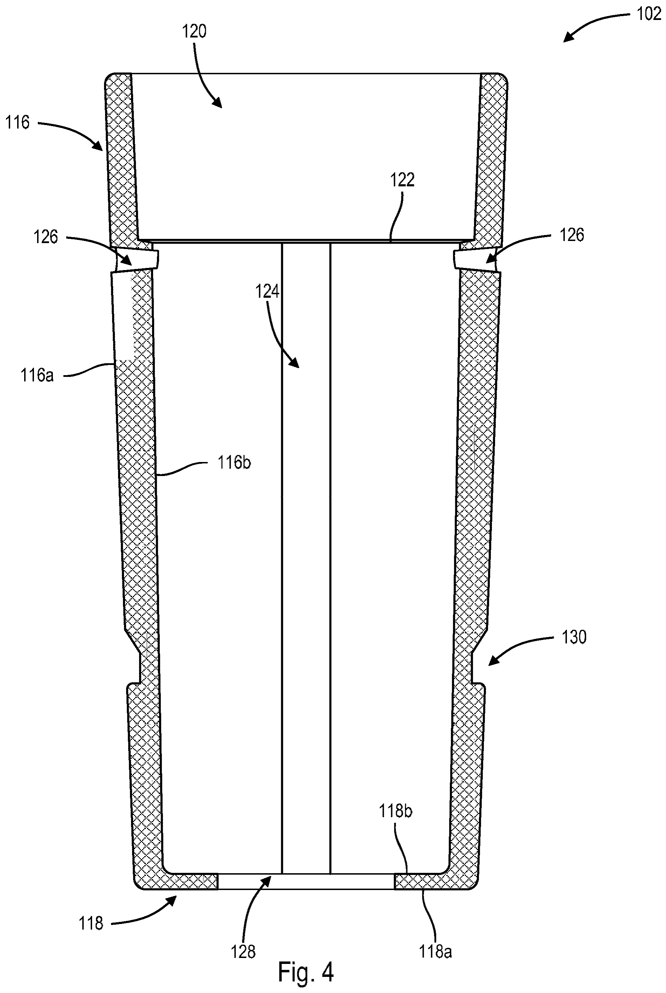

With particular reference to **Fig 4****,** the housing **102** includes a side wall **116** and a bottom wall **118.** The side wall **116** includes an outer surface **116a** and an opposed inner surface **116b.** The bottom wall **118** includes an outer surface **118a** and an opposed inner surface **118b.** The side wall **116** extends substantially vertically from the bottom wall **118.** The side wall **116** and the bottom wall **118** are generally cylindrical in shape and are concentric with one another relative to a longitudinal axis of the side wall **116.** The inner surface **116b** of the side wall **116** and the inner surface **118b** of the bottom wall **118** define an inner volume **120.** An upper portion of the inner surface **116b** of the side wall **116** is wider than a lower portion of the inner surface **116b** such that the inner surface **116b** defines a ledge **122.** The inner surface **116b** also defines a first and second groove **124** that are opposed from one another (only one of which is shown in the cross section in **Fig. 4****).** The grooves **124** extend between the ledge **122** and the inner surface **118b** of the bottom wall **118.**

The side wall **116** defines openings **126** that extend through the side wall **116.** That is, the openings **126** extend between the outer surface **116a** and the inner surface **116b** of the side wall **116.** The bottom wall **118** defines an aperture **128** that extends through the bottom wall **118.** That is, the aperture **128** extends between the outer surface **118a** and the inner surface **118b** of the bottom wall **118.** As will be discussed in further detail herein, when the lancet **100** is activated via engagement with the cartridge **12,** the needles of the lancet **100** extend through the aperture **128** for puncturing a subject's skin to draw a physiological sample. The outer surface **118** of the side wall **116** further defines an outer groove **130.** As will be discussed in further detail herein, a portion of the cartridge **12** engages with the groove **130** to couple the lancet **100** to the cartridge **12.**

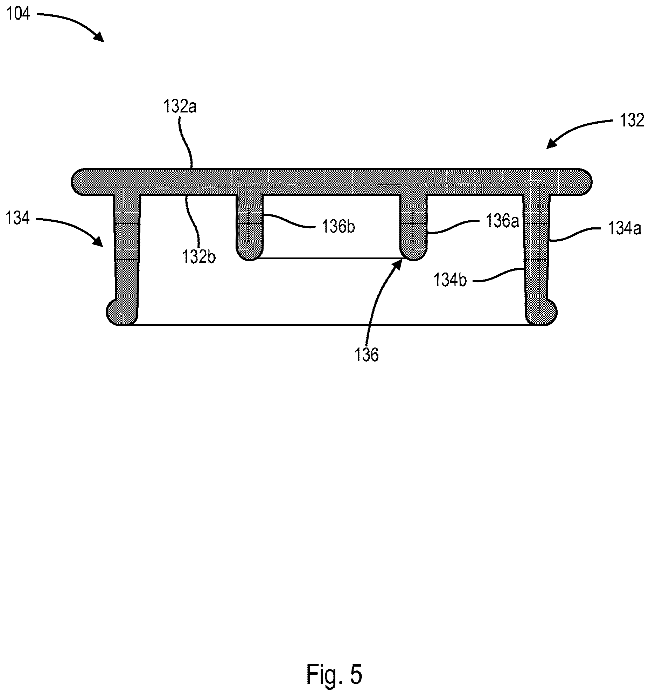

Referring now to **Fig. 5****, the** inner cap **104** is shown in accordance with an exemplary embodiment. The cap **104** includes a top wall **132** with an outer surface **132a** and an opposed inner surface **132b.** The cap **104** also includes a side wall **134** with an outer surface **134a** and an opposed inner surface **134b.** The top wall **132** extends substantially horizontally from and perpendicular to the side wall **134.** The side wall **134** extends substantially vertically from and perpendicular to the top wall **132.** The top wall **132** and the side wall **134** are generally circular in shape and are generally concentric with one another. The cap **104** also includes an inner cylinder **136** with an outer surface **136a** and an opposed inner surface **136b.** Inner cylinder **136** extends substantially vertically from and perpendicular to the top wall **132.** The inner cylinder **136** is generally concentric with the top wall **132** and the side wall **134.** The inner cylinder **136** extends substantially vertically from and perpendicular to the top wall **132.** The inner cylinder **136** is concentric with the top wall **132** and the side wall **134.**

When the cap **104** is coupled to the housing **102** the side wall **134** extends into the inner volume **120** of the housing **102** and at least a portion of the side wall **134** contacts the inner surface **116b** of the side wall **116** such that the cap **104** couples to the housing **102** via an interference fit.

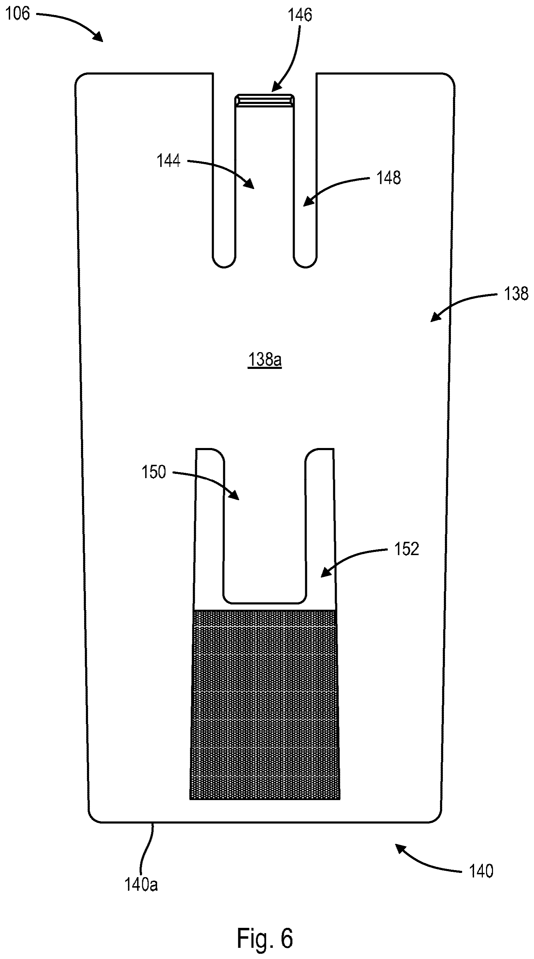

Referring now to **Figs. 6-8****,** the inner sleeve **106** is shown in accordance with an exemplary embodiment. The inner sleeve **106** includes a side wall **138** and a bottom wall **140.** The side wall **138** includes an outer surface 138a and an opposed inner surface **138b.** The bottom wall **140** includes an outer surface **140a** and an opposed inner surface **140b.** The side wall **138** extends substantially vertically from the bottom wall **140.** The side wall **138** and the bottom wall **140** are generally circular in shape and are concentric with one another. The inner surface **138b** of the side wall **138** and the inner surface **140b** of the bottom wall **140** define an inner volume **142.**

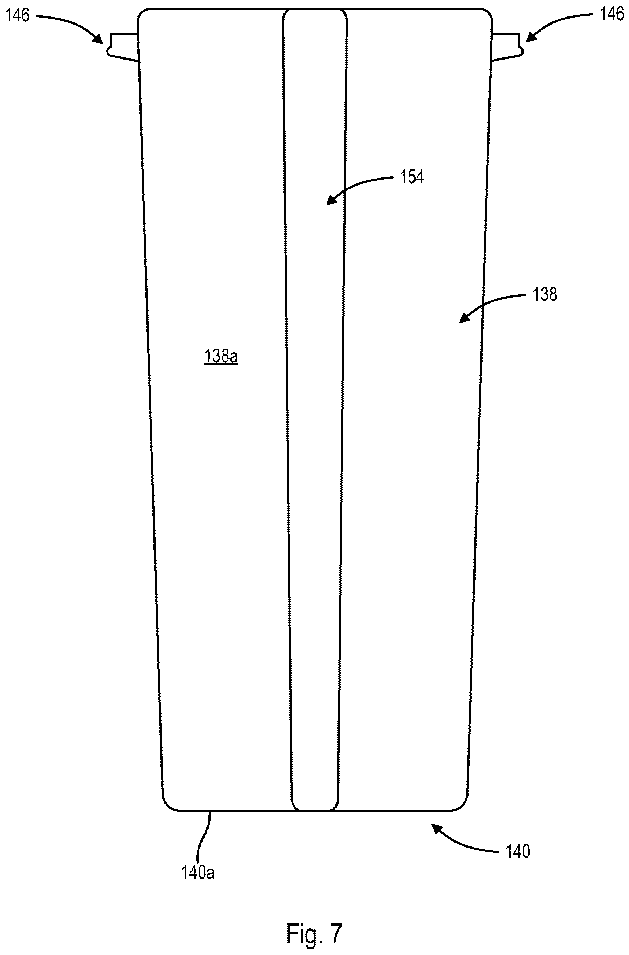

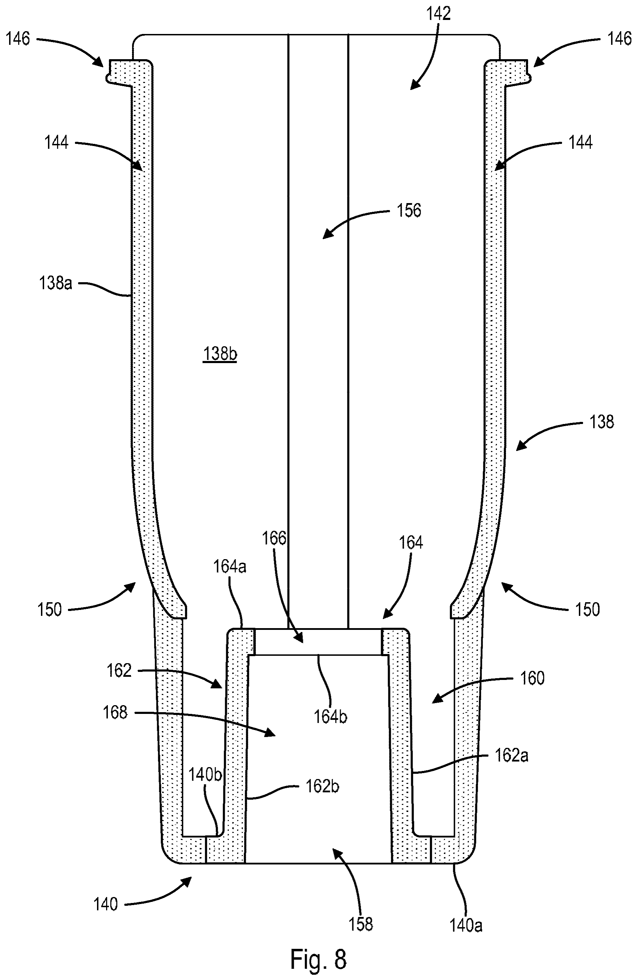

The side wall **138** defines a first and second deformable extension **144** that extend within a gap **148** of the side wall **138.** The extensions **144** each include a hook **146.** As will be discussed in further detail herein, the hooks **146** of the extensions **144** extend into the openings **126** of the housing **102** which retains the lancet **100** in the undeployed position. The side wall **138** further defines a first and second deformable tab **150** that extend within an opening **152** of the side wall **138.** The side wall **138** also includes a first and second protrusion **154** (only one of which is shown in **Fig. 7****)** that extend substantially horizontally from and perpendicular to the outer surface **138a** of the side wall **138.** When the inner sleeve **106** is disposed within the housing **102,** the protrusions **154** extend within the grooves **124** of the housing **102.** When the inner sleeve **106** is moving between positions (e.g., between an undeployed position to a deployed position), the grooves **124** and the protrusions **154** guide the vertical movement of the inner sleeve **106** while preventing a rotational or horizontal movement of the inner sleeve **106.** The inner surface **138b** of the side wall **138** further defines a first and second groove **156** that are opposed from one another (only one of which is shown in **Fig. 8****).** The grooves **156** extend substantially vertically from and perpendicular to the inner surface **140b** of the bottom wall **140.**

The inner sleeve **106** includes an opening **158** that extends through the bottom wall **140.** That is, the opening **158** extends between the outer surface **140a** and the inner surface **140b** of the bottom wall **140.** The inner sleeve **106** also includes a post **160** that is cylindrical in shape and is generally concentric with the opening **158.** The post **160** includes a side wall **162** and a top wall **164.** The side wall **162** extends substantially vertically from and perpendicular to the inner surface **140b** of the bottom wall **140.** The side wall **162** includes an outer surface **162a** and an opposed inner surface **162b.** The top wall **164** extends substantially horizontally between the side wall **162** and includes a top surface **164a** and an opposed bottom surface **164b.** The top wall **164** includes an opening **166** that extends through the top wall **164.** That is, the opening **166** extends between the top surface **164a** and the bottom surface **164b** of the top wall **164.** The inner surface **162b** of the side wall **162** and the bottom surface **164b** of the top wall **164** define an inner volume **168** of the post **160.** As will be discussed in further detail herein, the needles 110 extend through the opening **166** of the top wall **164,** through the inner volume **168,** and through the opening **158** of the bottom wall **140** when in the deployed position.

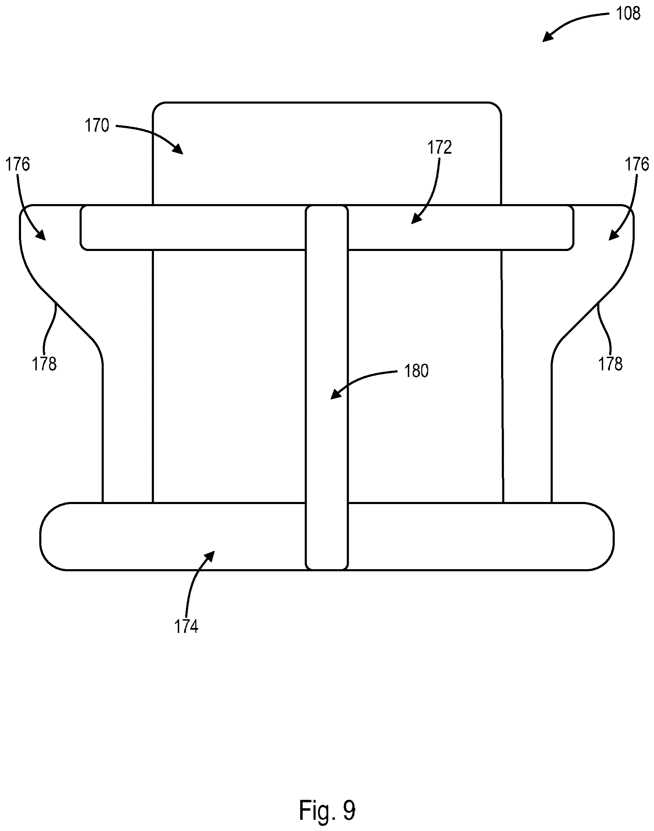

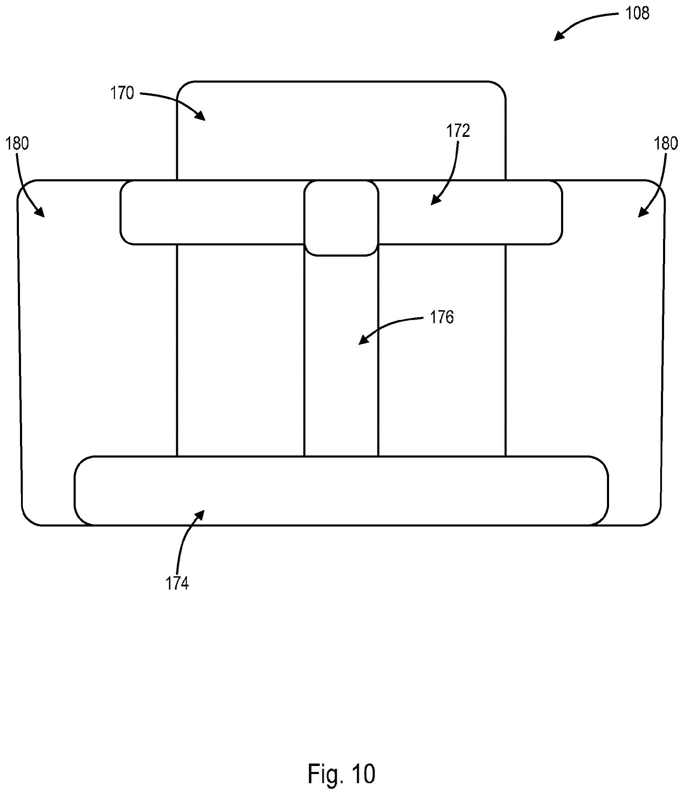

Referring now to **Figs. 9** **and** **10****,** the needle platform **108** is shown in accordance with an exemplary embodiment. The needle platform **108** includes a cylindrical body **170,** an upper circular extension **172** and a lower circular extension **174.** The upper circular extension **172** and the lower circular extension **174** extend substantially horizontally from and perpendicular to the cylindrical body **170.** As will be discussed in further detail herein, the upper circular extension **172** serves as a platform for the injection spring **112.** The needle platform **108** further includes a first and second curved extension **176.** The curved extensions **176** extend substantially horizontally from and perpendicular to the cylindrical body **170** and extend substantially vertically between the upper circular extension **172** and the lower circular extension **174.** Each curved extension **176** includes an angled surface **178.** As will be discussed in further detail herein, when in the undeployed position, the angled surfaces **178** contact the hooks **146** of the inner sleeve **106** which prevents the needle platform **108** from transitioning to the deployed position. The needle platform **108** further includes a first and second a projection **180.** The first and second projection **180** are generally rectangular in shape, extend substantially horizontally from and perpendicular to the cylindrical body **170,** and extend substantially vertically between the upper circular extension **172** and the lower circular extension **174.** When the needle platform **108** is disposed within the inner sleeve **106,** the projections **180** extend into the grooves **156** which guides the vertical movement of the needle platform **108** while preventing a rotational or horizontal movement of the needle platform **108** needle platform **108.**

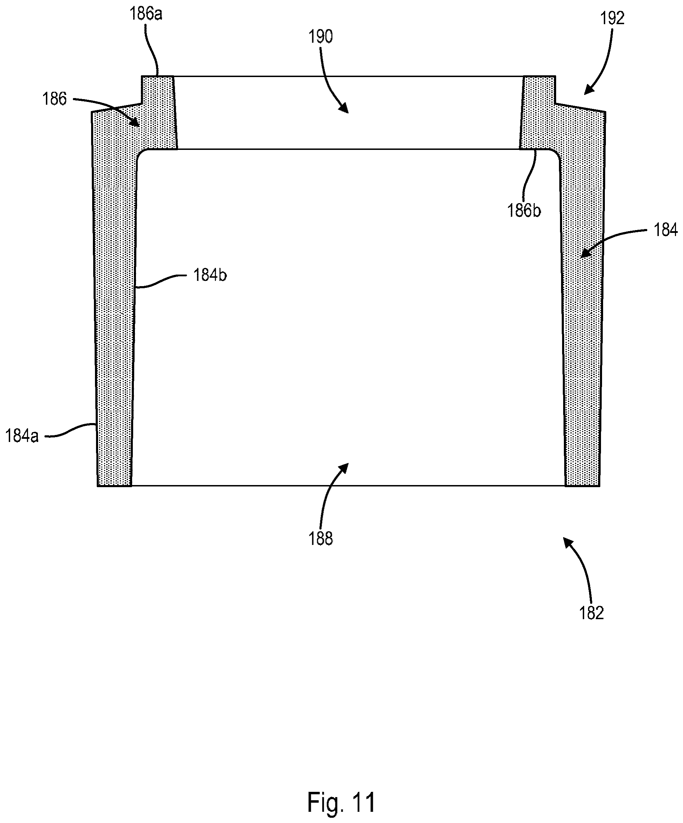

As depicted in **Fig. 3****,** the lancet **100** further includes a spring sleeve **182.** With particular reference to **Fig. 11****,** the spring sleeve **182** is shown in accordance with an exemplary embodiment. The spring sleeve **182** is cylindrical in shape and includes a side wall **184** and a top wall **186.** The side wall **184** includes an outer surface **184a** and an opposed inner surface **184b** and the top wall **186** includes a top surface **186a** and an opposed bottom surface **186b.** Th inner surface **184b** of the side wall **184** and the bottom surface **186b** of the top wall **186** define an inner volume **188** of the spring sleeve **182.** The side wall **184** and the top wall **186** are shaped and dimensioned such that the spring sleeve **182** retains the retraction spring **114** within the inner volume **188.** The top wall **186** includes an opening **190** that extends therethrough. That is, the opening **190** extends between the top surface **186a** and the bottom surface **186b** of the top wall **186.** The opening **190** of the top wall **186** is in communication with the inner volume **188.** Since the inner volume **188** is open, the needles **110** may extend through spring sleeve **182** via the opening **190.** The top surface **184a** defines a ledge **192** that extends circumferentially around the spring sleeve **182.** As will be discussed in further detail herein, when the lancet **100** is in the undeployed position, the tabs **150** contact the ledge **192.**

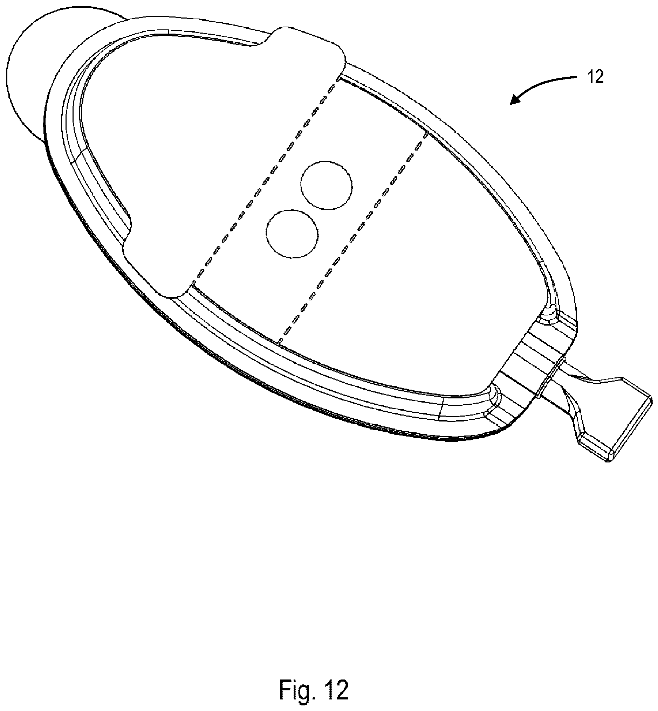

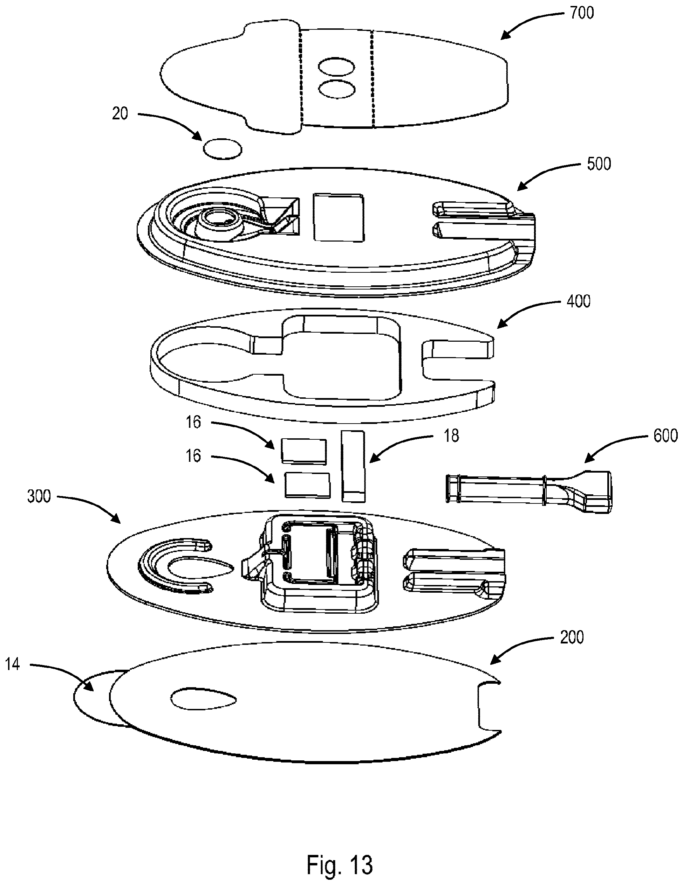

Referring to **Figs. 12** **and** **13****,** the cartridge **12** is shown in accordance with an exemplary embodiment. As will be discussed in further detail herein, the cartridge **12** is formed of a plurality of layers of suitable materials including, but not limited to, polymeric materials (e.g., polyolefins, polyethylene terephthalate (PET), polyurethanes, polynorbomenes, polyethers, polyacrylates, polyamides (Polyether block amide also referred to as Pebax^{®}), polysiloxanes, polyether amides, polyether esters, trans-polyisoprenes, polymethyl methacrylates (PMMA), cross-linked trans-polyoctylenes, cross-linked polyethylenes, cross-linked polyisoprenes, cross-linked polycyclooctenes, inorganic-organic hybrid polymers, co-polymer blends with polyethylene and Kraton^{®}, styrene-butadiene co-polymers, urethane-butadiene co-polymers, polycaprolactone or oligo caprolactone co-polymers, polylactic acid (PLLA) or polylactide (PL/DLA) co-polymers, PLLA-polyglycolic acid (PGA) co-polymers, photocross linkable polymers, etc.). In some embodiments, all or a portion of each material layer may be formed of poly(dimethylsiloxane) (PDMS) to allow visibility of components disposed within the cartridge **12.** While the various layers of material that form the cartridge **12** are said to be layers of polymeric material, it is understood that the layers of material that form the cartridge **12** may be formed of any suitable material (e.g., metal, metal alloy, etc.). As will be discussed in further detail herein, the layers can be stacked and coupled to one another to form the cartridge **12.** In some embodiments, the layers of the cartridge **12** may be formed of aluminum or other suitable metals. In certain embodiments, the layers of the cartridge **12** may be formed of a polymeric coated with aluminum film or another suitable metal film. In some embodiments, the layers of material, including the channels disclosed herein, may be thermoformed.

The cartridge **12** can include an adhesive layer **200,** a bottom layer **300** of polymeric material, a desiccant **400,** a top layer **500** of polymeric material, and a moveable vacuum pin **600** that is disposed between the bottom layer **300** and the top layer **500.** The desiccant can be formed in the space between the top layer 500 and the bottom layer 300. The adhesive layer 200 can be formed of a pressure sensitive adhesive (PSA). The vacuum pin 600 can be thermoformed and laser welded. The vacuum pin can also be made of a polymeric material that is 3D printed or injection molded or compression molded or casting. The cartridge **12** further includes an adhesive cover **700** that covers the top surface of the top layer **500** and a protective liner **14** (e.g., formed of paper) that covers the adhesive layer **200.** The protective liner **14** protects the adhesive layer **200** before the cartridge **12** is attached to the skin of a subject. When the adhesive layer **200** is pressed against the user's skin, the PSA can create an airtight seal that can allow the vacuum pin to work more effectively.

As further depicted in **Fig. 13****, the** cartridge **12** also includes a first and second collection pad **16** that are configured to absorb a physiological sample, a hydrophobic foam **18,** and a pierceable foil **20** that seals a portion of the cartridge **12.** The collection pad 16 can include a DBS/Whatman paper to absorb and collect the physiological sample, as in the CF12 from Cytiva.

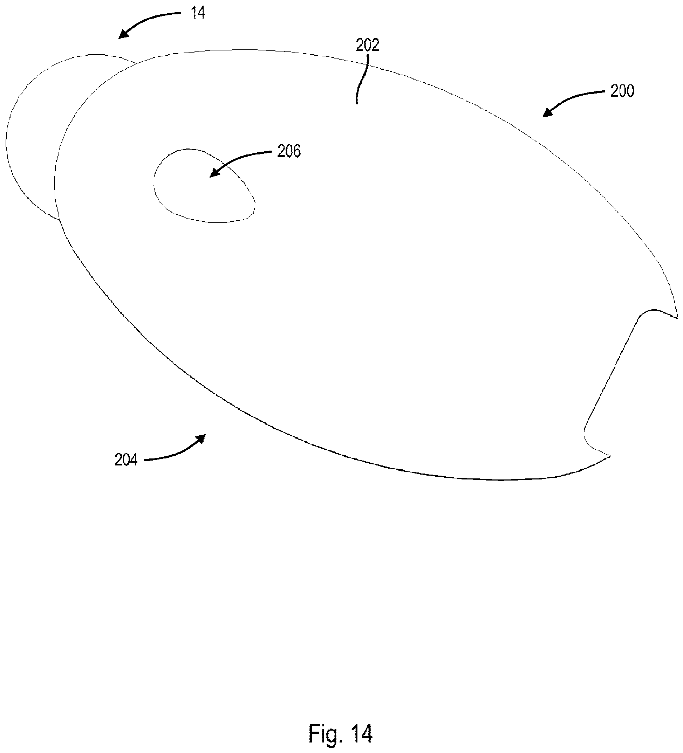

As depicted in **Fig. 14****,** the adhesive layer **200** includes a top surface **202** and an opposed bottom surface **204.** The adhesive layer **200** further includes a sample well opening **206** which extends through the adhesive layer **200,** but not necessarily through the liner **14.** That is, the sample well opening **206** extends between the top surface **202** and the bottom surface **204.** **Fig. 14** shows the sample well opening having a teardrop shape, but the sample well opening can also have a circular shape in order to reduce the distance between the sample well opening **206** and the needles **110.** The adhesive layer **200** has a similar shape and dimension as the bottom layer **300** such that the adhesive layer **200** covers the bottom layer **300.** The protective liner **14** covers the adhesive layer **200** and includes a pull tab which allows a user to remove the protective liner **14** from the cartridge **12** thereby allowing a user to attach the cartridge **12** to the skin of a subject.

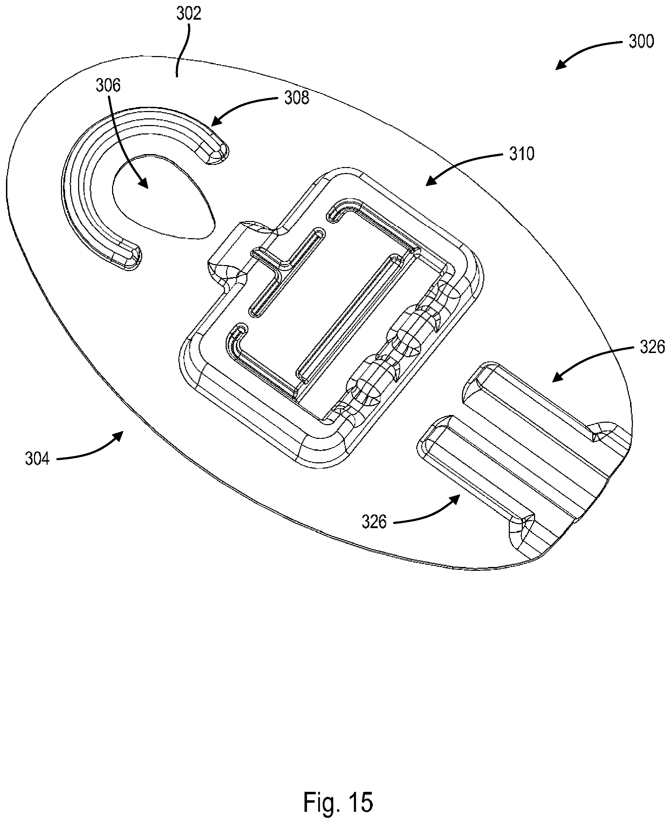

The bottom layer **300 (****Fig. 15****)** can have a thickness in a range of about 0.4 mm to about 0.6 mm (e.g., 0.5 mm) and includes a top surface **302** and an opposed bottom surface **304.** The bottom layer **300** also includes a sample well opening **306** that extends through the bottom layer **300.** That is, the sample well opening **306** extends between the top surface **302** and the bottom surface **304.** The bottom layer **300** further includes a rim **308** that extends substantially vertically from and perpendicular to the top surface **302.** The rim **308** at least partially surrounds the sample well opening **306.** As will be discussed in further detail herein, the rim **308** aids in securing the bottom layer **300** to the top layer **500.**

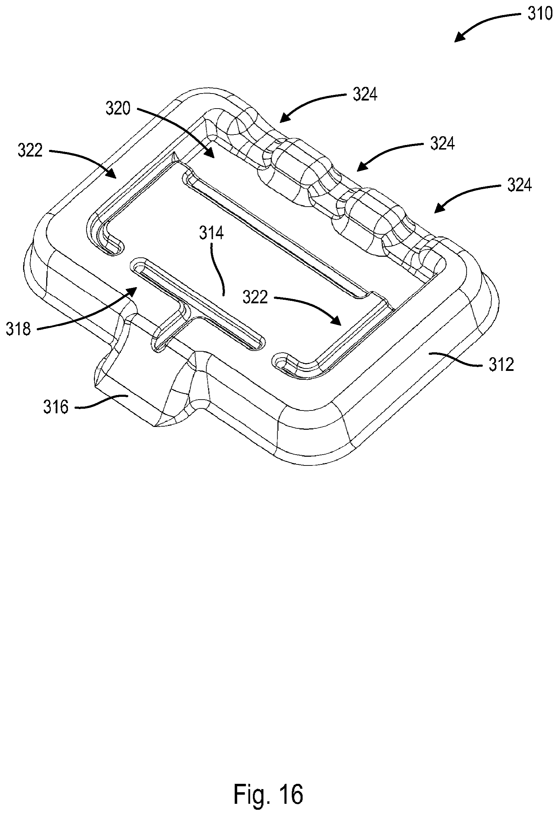

The bottom layer **300** further includes a collection pad support **310 (****Fig. 16****).** The collection pad support is generally rectangular and includes a side wall **312** and a top wall **314.** The side wall **312** extends substantially vertically from and perpendicular to the top surface **302.** The top wall **314** extends substantially horizontally from and between the side wall **312.** The collection pad support **310** further includes a slanted wall **316** that extends between the top surface **302** and the top wall **314.** The top wall **314** of the collection pad support **310** includes a T-shaped groove **318.** As will be discussed in further detail herein, a physiological sample drawn from a subject travels along a portion of the slanted wall **316** to the T-shaped groove **318.** The top wall **314** further includes a hydrophobic foam receptacle **320,** opposing side grooves **322,** and three notches **324.** The side grooves **322** and the three notches **324** are in open communication with the hydrophobic foam receptacle **320** that retains the hydrophobic foam **18.** The bottom layer 300 further includes two vacuum pin supports **326 (****Fig. 15****)** that extend substantially vertically from and perpendicular to the top surface **302.** The vacuum pin supports **326** are shaped to accept at least a portion of the vacuum pin **600.**

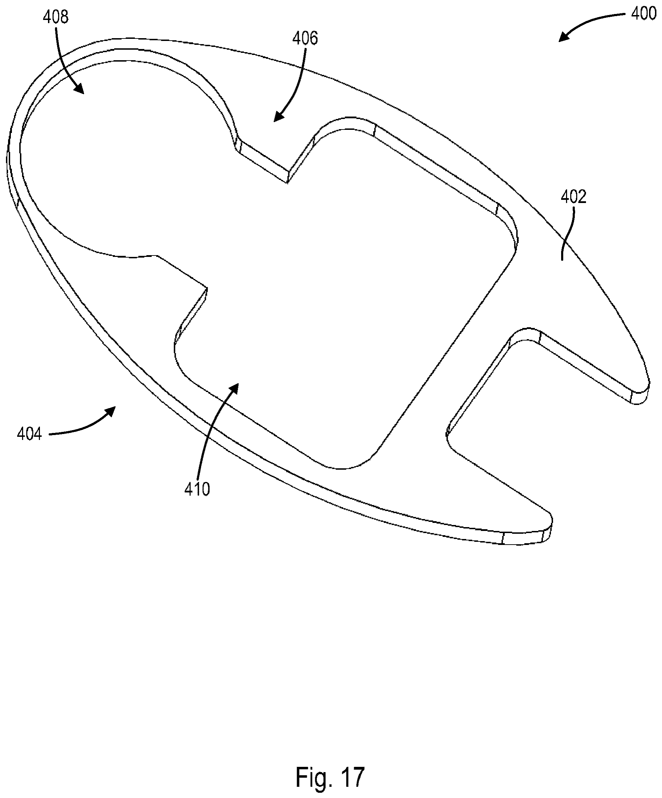

Referring now to **Fig. 17****,** the desiccant **400** is shown in accordance with an exemplary embodiment. The desiccant **400** can have a thickness of about 3.5 mm and includes a top surface **402** and an opposed bottom surface **404.** The desiccant **400** includes an opening **406** that extends through the desiccant **400.** That is, the opening **406** extends between the top surface **402** and the bottom surface **404.** The opening **402** includes a rounded portion **408** and a rectangular portion **410.** When the cartridge **12** is assembled, the bottom layer **300** and the top layer **500** define an inner void of the cartridge **12** that is shaped to accept and accommodate the desiccant **400.** Furthermore, the rounded portion **408** of the opening **402** has a similar shape and dimension as the rim **308** and the rectangular portion **410** of the opening **402** has a similar shape and dimension as the side wall **312** of the collection pad support 310 such that desiccant **400** contacts the rim 308 and the collection pad support **310.** This contact prevents the desiccant **400** from moving within the cartridge **12.** The desiccant **400** also includes an indentation **412 (show in** **Fig. 17****).** The indentation **412** is formed such that the desiccant **400** does not contact the vacuum pin supports 326 when the cartridge **12** is assembled. The desiccant **400** is formed of a desiccant material (e.g., silica gel, calcium chloride, molecular sieve, activated carbon, activated alumina, aluminum oxide, moisture absorbing polymers, desiccant-infused absorbent pads, etc.). As such, the desiccant **400** dries substances that are in open communication with it. As will be discussed in further detail herein, the desiccant **400** dries a physiological sample that has been absorbed by a collection pad **16** of the cartridge **12.**

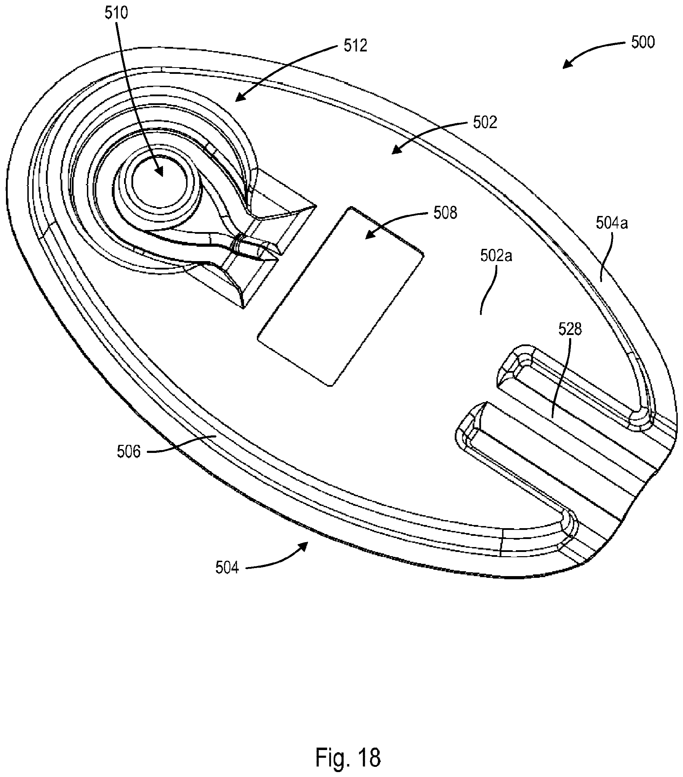

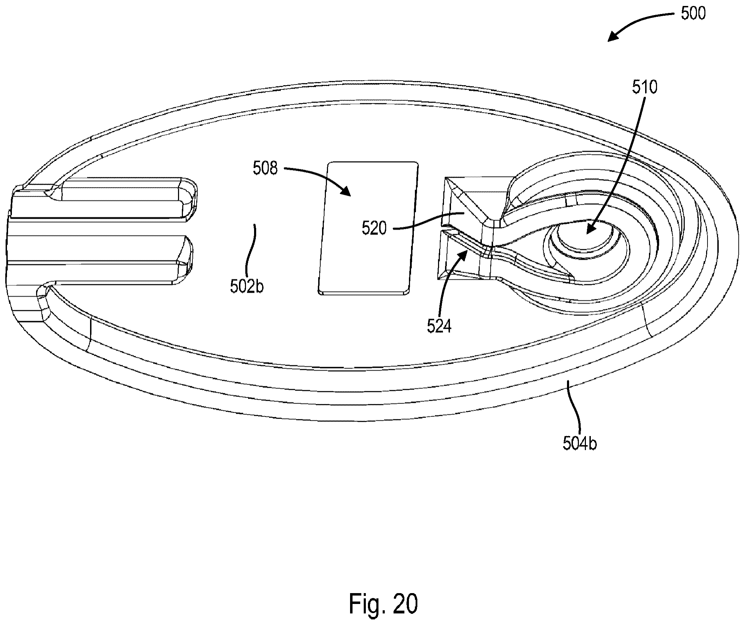

With reference to **Figs. 18-20****,** the top layer **500** is shown in accordance with an exemplary embodiment. The top layer **500** can have a thickness in a range of about 0.25 mm to about 3.0 mm (e.g., 0.75 mm) and includes a top wall **502,** a bottom wall **504,** and a side wall **506** that extends between the top wall **502** and the bottom wall **504.** The top wall **502** includes an outer surface **502a** and an opposed inner surface **502b.** The bottom wall **504** includes a top surface **504a** and an opposed bottom surface **504b.** The side wall **506** extends substantially vertically from and perpendicular between the top wall **502** and the bottom wall **504.** When the cartridge **12** is assembled, the bottom surface **504b** of the bottom wall **504** is affixed to the top surface **302** of the bottom layer **300.** The top layer **500** may be affixed to the bottom layer **300** by laser welding, heat sealing, thermal bonding, pressure sensitive adhesive, heat induction, adhesive bonding, ultrasonic welding diffusion bonding, or mechanical locking. Furthermore, the top wall **502,** the side wall **504,** and the top surface **302** of the bottom layer **300** define an inner volume of the cartridge **12** in which the desiccant **400** is disposed.

The top layer **500** further includes a sample collection pad opening **508** that extends through the top wall **502.** That is, the opening **508** extends between the outer surface **502a** and the inner surface **502b** of the top wall **502.** As will be discussed in further detail herein, the sample collection pad opening **508** is shaped and dimensioned to accommodate two sample collection pads **16.** The top layer **500** also includes a needle aperture **510** that extends through the bottom wall **504.** That is, the needle aperture **510** extends between the top surface **504a** and the bottom surface **504b** of the bottom wall **504.** When the cartridge **12** is assembled, the needle aperture **510** is aligned with the sample well opening **306.** The needle aperture **510 can** be made smaller than the sample well opening **206** in order to hold the skin closer to the lancet, thereby helping prevent the skin from excessive tenting caused by blade penetration.

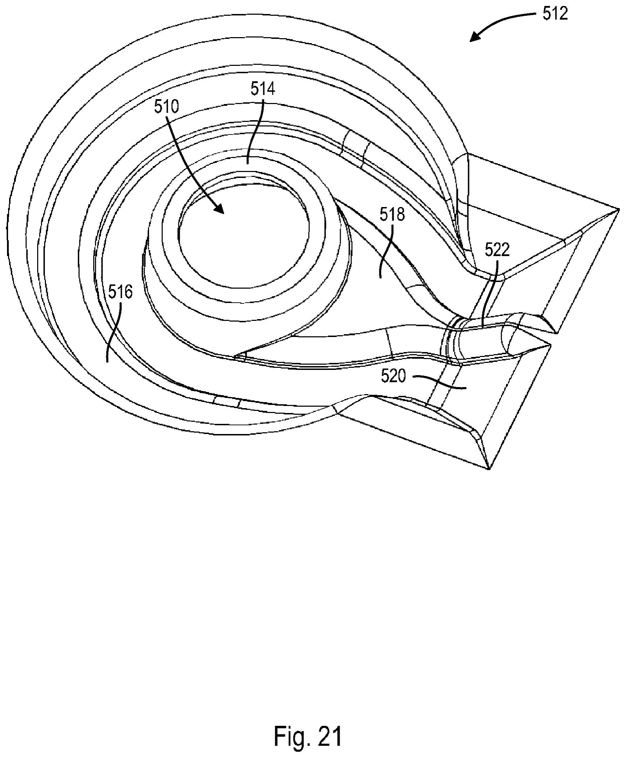

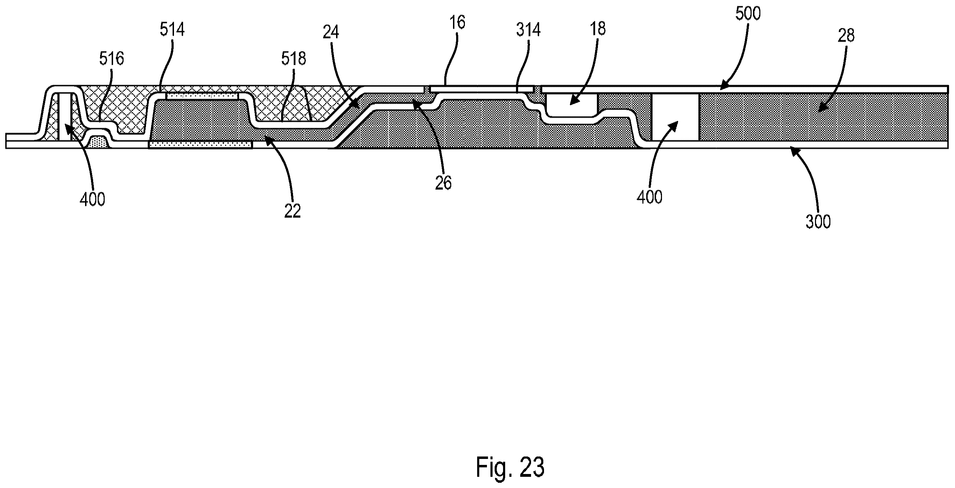

The top layer **500** further includes a lancet receiving element **512 (****Fig. 21****)** that extends about the needle aperture **510.** The lancet receiving element 512 includes a circular extension **514** that extends vertically from and perpendicular to the top surface 504a of the bottom wall **504.** The circular extension **514** surrounds and is substantially concentric with the needle aperture **510.** As will be discussed in further detail herein, the circular extension **514** actuates the lancet **100** to draw a physiological sample from a subject. The lancet receiving element **512** further includes a rounded wall **516** that extends substantially vertically from and perpendicular to the top surface **504a** of the bottom wall **504.** The lancet receiving element **512** further includes a sample well wall **518** that extends substantially vertically from and perpendicular to the top surface **504a** of the bottom wall **504.** The sample well wall **518** also extends substantially horizontally form and perpendicular to the circular extension **514.** When the cartridge **12** is assembled, the sample well wall **518** and the sample well opening **306** define a physiological sample well **22 (****Fig. 23****)** that is in open communication with the needle aperture **510.**

The top layer **500** further includes a slanted wall **520** that extends between the top wall **502** and the bottom wall **504.** The slanted wall **520** includes a semicircular extension **522** that defines a groove **524** of the slanted wall **520.** The slanted wall **316** and the slanted wall **520** have substantially the same slope such that when the cartridge **12** is assembled, the slanted walls **520** and **316** contact and are substantially parallel to one another. The groove **524** and the slanted wall **316** define a first channel **24** of the cartridge **12 (****Fig. 23****).** The first channel **24** extends from and is in open communication with the sample well **22.** Furthermore, the T-shaped groove **318** and the top wall **502** define a second channel **26** that is in open communication with the first channel **24.** As will be discussed in further detail herein, the first channel **24** and the second channel **26** carry a physiological sample from the sample well **22** to the collection pads **16.** The first and second channels 24 and 26 can have a diameter in the range of approximately 0.5 mm - 1.5 mm.

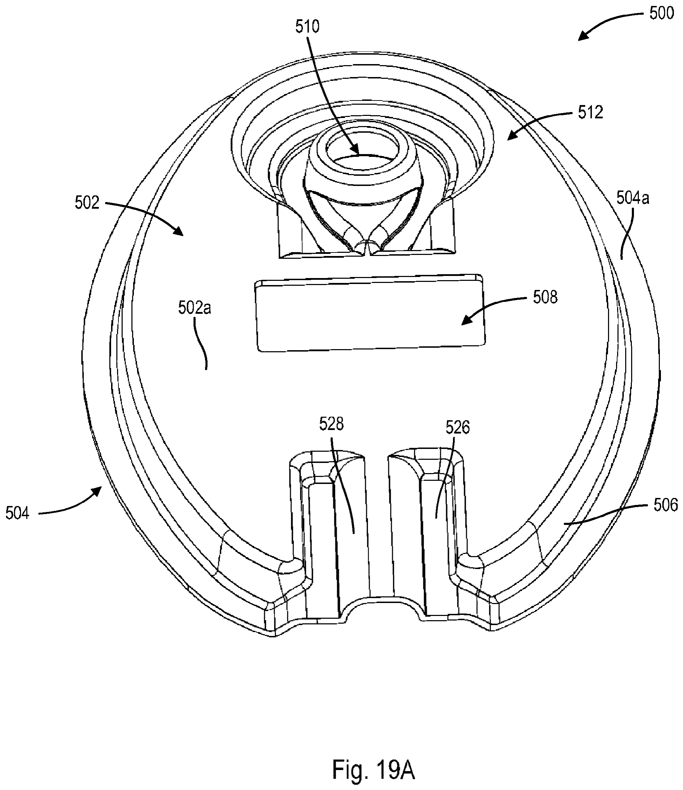

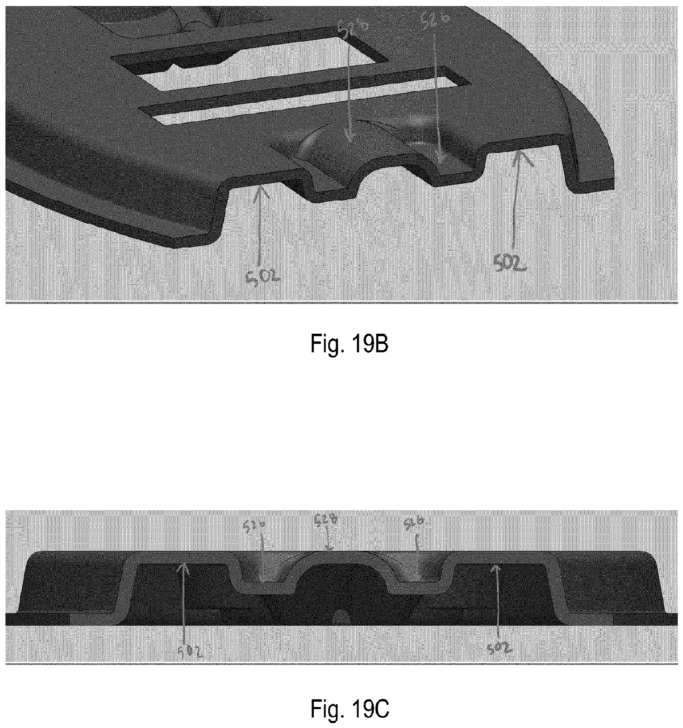

The top layer **500** also includes a U-shaped wall **528** and flat sections **526** adjacent the U-shaped wall 528 (see **Figs. 19A - 19C****).** The edge portions of the U-shaped wall **528** extend substantially vertically from the bottom wall 502 and the flat sections **526** extend vertically from the flat sections **526.** The U-shaped wall **528** has a similar shape and dimension as the vacuum pin supports **326** such that when the cartridge **12** is assembled, the vacuum pin supports **326** contact the U-shaped wall **528.** The U-shaped wall **528** and the vacuum pin supports **326** define a vacuum pin receptacle **28 (****Fig. 23****)** that is shaped and dimensioned to accept a portion of the vacuum pin **600.**

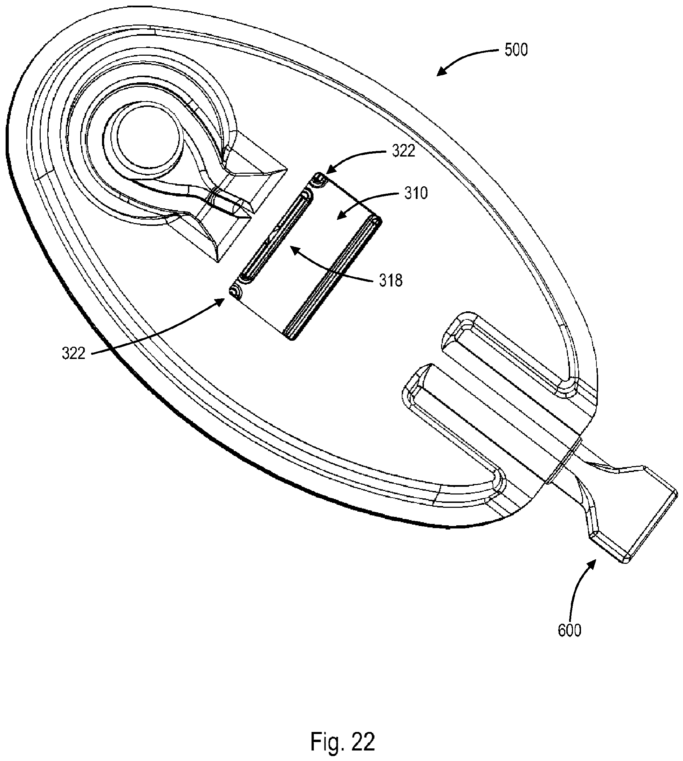

As depicted in **Fig. 22****,** when the cartridge **12** is assembled, the collection pad support **310** is aligned with the sample collection pad opening **508** such that the collection pads **16** disposed on the top wall **314** of the collection pad support **310** extend through the sample collection pad opening **508.** The collection pads **16** are positioned on the collection pad support **310** such that the collection pads **16** are positioned above a portion of the T-shaped groove **318** and a portion of a side groove **322.**

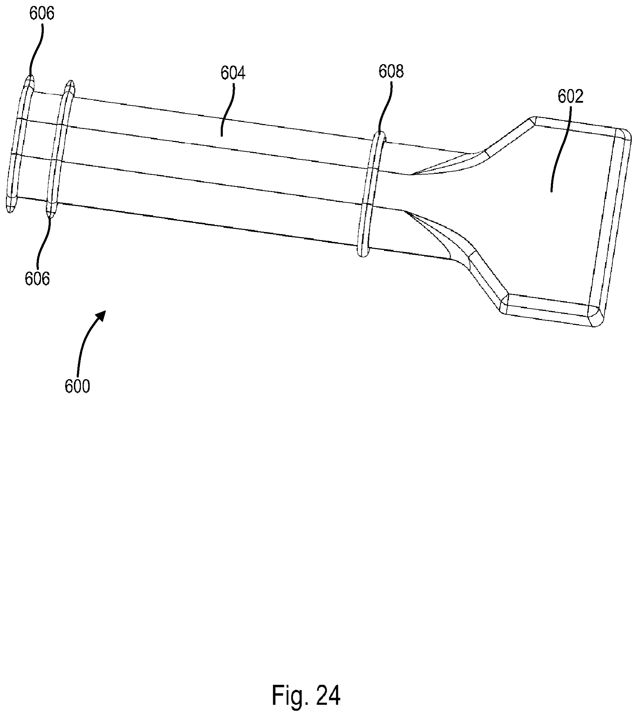

Referring now to **Fig. 24****,** the vacuum pin **600** is shown in accordance with an exemplary embodiment. In this embodiment, the vacuum pin **600** includes a handle **602** and a neck **604** that extends from the handle **602.** The vacuum pin **600** also includes a plurality of ridges **606** that extend vertically from and around the neck **604.** In some embodiments, the ridges **606** may be formed of an elastomeric material. The vacuum pin **600** also includes a rim **608** that extends substantially vertically from and around the neck **604.** The rim **608** has a wider diameter than the ridges **606** and prevents a user from pushing the vacuum pin **600** farther into the cartridge **12** as the rim **608** contacts the bottom layer **200** and the top layer **500** when pushed. When the vacuum pin **600** is disposed within the cartridge **12,** the ridges **606** contact the surfaces of the vacuum pin receptacle **28** (e.g., the bottom surface **302,** the vacuum pin supports **326,** and the U-shaped wall **528)** thereby providing an airtight seal between the vacuum pin **600** and the vacuum pin receptacle **28.**

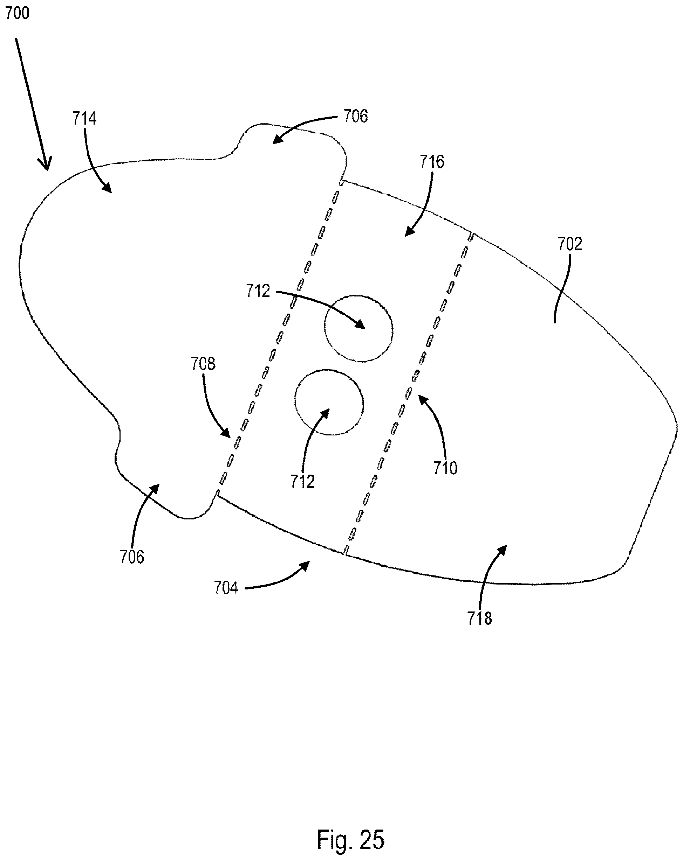

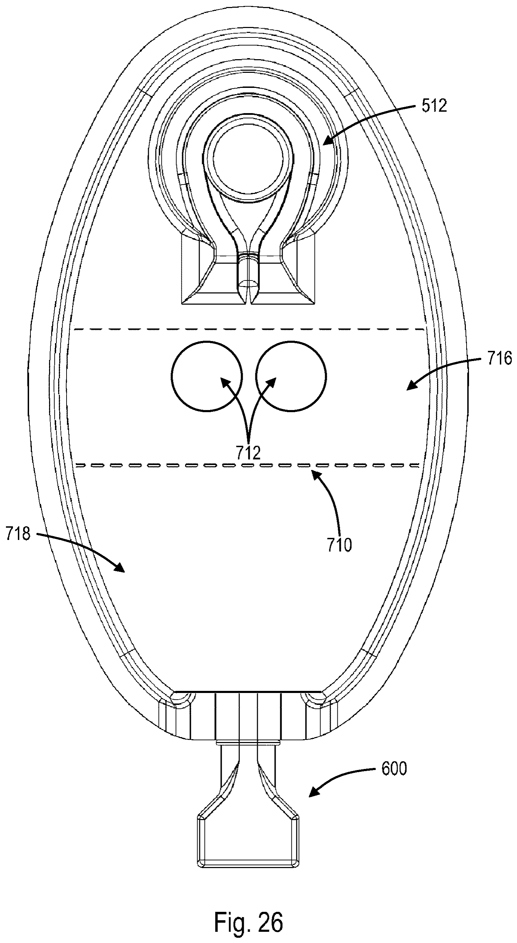

With reference to **Fig. 25****,** the adhesive cover **700** includes a top surface **702,** an opposed bottom surface **704,** pull tabs **706,** a first perforation **708,** a second perforation **710,** and viewing apertures **712.** The bottom surface **704** includes an adhesive that affixes the bottom surface **704** to the top wall **502** of the top layer **500.** The viewing apertures **712** are aligned with the collection pads **16** and may be covered with a transparent film that allows a user of the dermal patch system **10** to view the collection pads **16** in order to visually confirm blood collection. As depicted in **Fig. 26****,** user can pull a pull tab **706** to separate first portion **714** of the adhesive cover **700** from a second portion **716** and a third portion **718** of the adhesive cover **700** by ripping the adhesive cover **700** at the first perforation **708** to expose the lancet receiving element **512. A** medical professional can further rip the adhesive cover **700** at the second perforation **710** to remove the second portion **716** from the third portion **718** of the adhesive cover **700** to expose the collection pads **16.** Serial numbers as well as QR codes can be printed on the adhesive cover 700 to identify the cartridge in order to preserve the chain of custody.

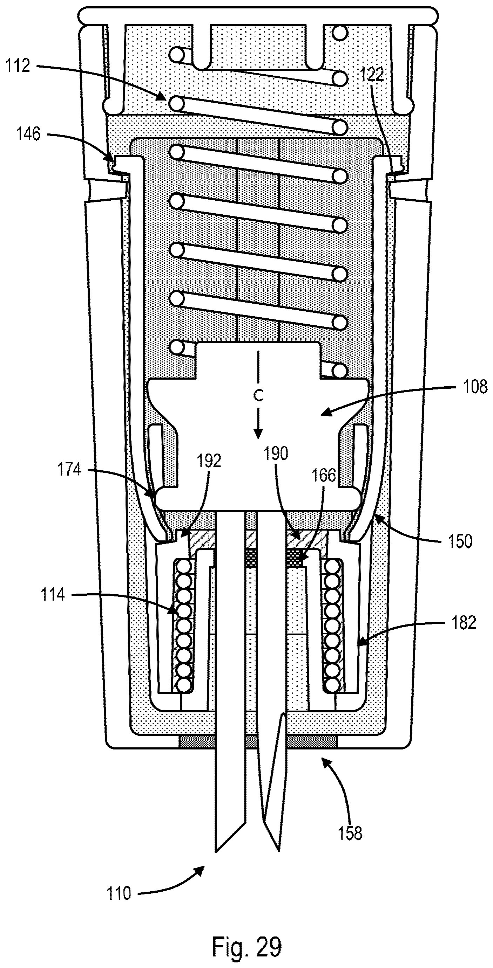

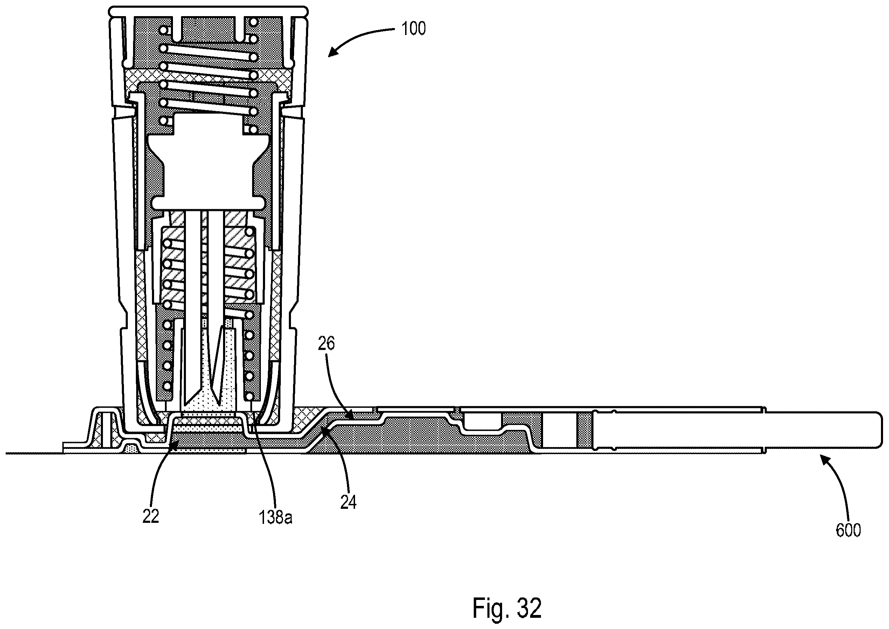

The lancet **100** is moveable between an undeployed position **(****Fig. 1****),** a deployed position **(****Fig. 29****),** and a retracted position **(****Fig. 32****).**

In the undeployed position, the hooks **146** of the inner sleeve **106** extend through the openings **126** of the housing **102** which retains the lancet **100** in the undeployed position and prevents the lancet **100** from moving to the deployed position. In this position, the extensions **144** are compressed inward toward the center of the lancet **100** which allows the needle platform **108** to rest upon the inner sleeve **106** which prevents the needle platform from moving vertically downward and into the deployed position. Specifically, the angled surface **178** of the extensions **176** of the needle platform **108** contacts rests upon the extensions **144.**

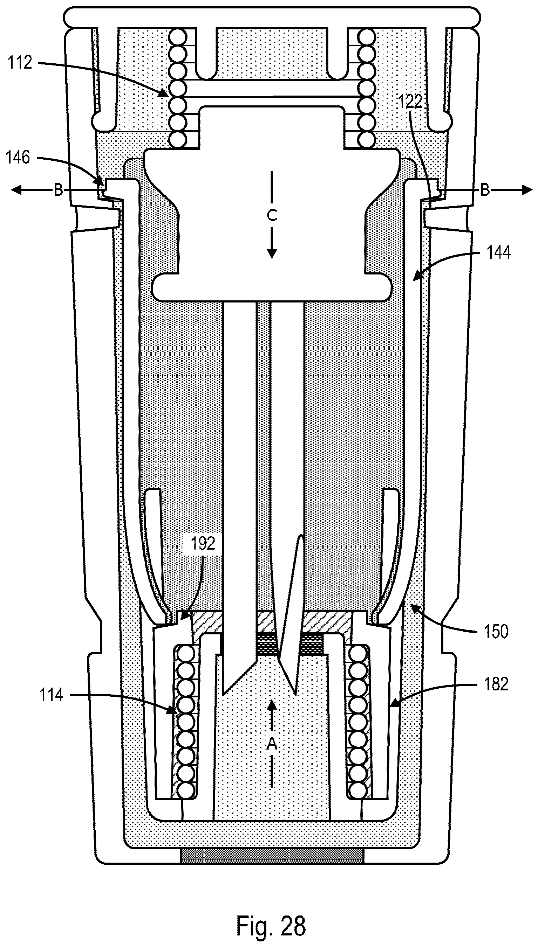

Furthermore, the injection spring **112** extends around the body **170** of the needle platform **108** and the outer surface **136a** of the inner cylinder **136.** The injection spring **112** extends substantially vertically between the inner surface **132b** of the top wall **132** of the cap **104** and the upper circular extension **172** of the needle platform **108.** In the undeployed position, the injection spring **112** is compressed between the top wall **132** of the cap **104** and the upper circular extension **172** of the needle platform **108.** The retraction spring **114** extends around the post **160** of the inner sleeve **106.** The retraction spring **114** extends substantially vertically between the bottom wall **140** of the inner sleeve **106** and the top wall **186** of the spring sleeve **182.** That is, the retraction spring **114** extends substantially vertically between the inner surface **140b** of the bottom wall **140** of the inner sleeve **106** and the inner surface **186b** of the top wall **186** of **the** spring sleeve **182.** When the lancet **100** is in the undeployed position, the retraction spring **114** is compressed between the bottom wall **140** of the inner sleeve **106** and the top wall **186** of the spring sleeve **182,** as shown in **Fig. 28****.** Furthermore, in the undeployed position, the deformable tabs **150** contact the ledge **192** of the spring sleeve **182** which prevents the retraction spring from expanding and moving the spring sleeve **182.**

After a user removes the first portion **714** of the adhesive cover **700** to expose the lancet receiving element **512,** the lancet **100** can be engaged with the cartridge **12** by pushing the lancet **100** into the lancet receiving element **512.** This causes the needles **110** of the lancet **100** to automatically move from an undeployed position to a deployed position and automatically from the deployed position to a retracted position.

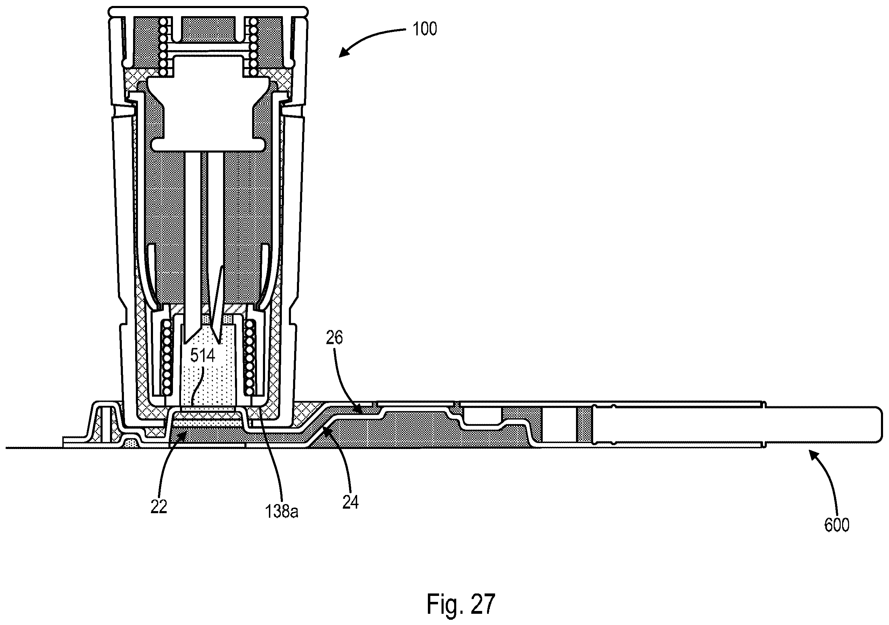

As depicted in **Figs. 27****,** **29****, and** **31****,** when the lancet **100** engages with the cartridge **12,** the bottom wall **140** rests upon the rounded wall **516** and the sample well wall **518** (see Fig. **21****).** Furthermore, upon engagement, the circular extension **514** of the lancet receiving element **512** extends through the aperture **128** of the housing **102** and contacts the bottom wall **140** of the inner sleeve **106.** Specifically, the top surface of the circular extension **514** contacts the outer surface **140a** of bottom wall **140** which forces the inner sleeve **106** to move vertically upward in the direction of arrow **A** within the housing **102.** That is, the movement of the inner sleeve **106** is activated automatically when the profile of the lancet **100** (i.e., the bottom wall **120** of the lancet **100)** interfaces with the profile of the cartridge **12** (i.e., circular extensions **514** of the cartridge **12).** As previously discussed herein, grooves **124** of the housing **102** and the protrusions **154** of the inner sleeve **106** guide the vertical movement of the inner sleeve **106.** The vertical movement of the inner sleeve **106** causes the hooks **146** to disengage from the openings **126** and causes the extensions **144** to extend vertically above and rest upon the ledge **122.** In his position, the extensions **144** decompress and expand outwardly in the direction of arrow **B.**

When the extensions **144** expand, the needle platform **108** no longer rests upon the extensions **144** which allows the needle platform **108** to move vertically downward in the direction of arrow **C.** As the needle platform **108** moves downward, the injection spring **112** is allowed to expand and the force applied by the injection spring **112** causes the needle platform **108** (and therefore the needles **110)** to travel with a force that is sufficient to cause the needles **110** to puncture the skin of a subject wearing the dermal patch system **10.** The injection spring **112** causes the needles **110** to pierce the foil **20** and to extend through the spring sleeve **182** via the opening **190,** through the post **160** via the opening **166,** through the opening **158** of the inner sleeve **106** and exit the housing **102** via the aperture **128** and pass through the cartridge **12** via the well opening **306** and the needle aperture **510.** Stated another way, the injection spring **112** moves the needles **110** to the deployed position. Accordingly, the lancet **100** can only be actuated when the profile/configuration of the lancet **100** engages with a matching profile/configuration of the cartridge **12.** Therefore, the lancet **100** cannot be actuated by simply pressing the lancet against the skin, thereby preventing unsafe deployment of the needles in the lancet **100.**

The parameters of the injection spring **112** can be selected to create the desired injection velocity, penetration force and penetration depth. The parameters of the retraction spring **114** can be selected to create the desired retraction depth within the lancet when the needles are fully retracted to meet desired safety standards (e.g., ISO standards). For example, the force of the injection spring **112** can be set in the range of approximately 13N to approximately 18N. Using an injection spring with these spring forces can create an injection velocity range of approximately 10 m/s to approximately 13 m/s. The force of the retraction spring **114** can be set in the range of approximately 25 N to approximately 32 N, preferably at 28N. Using a retraction spring with these spring forces can create a minimum retraction depth of approximately 3.5 mm to approximately 1.5 mm.

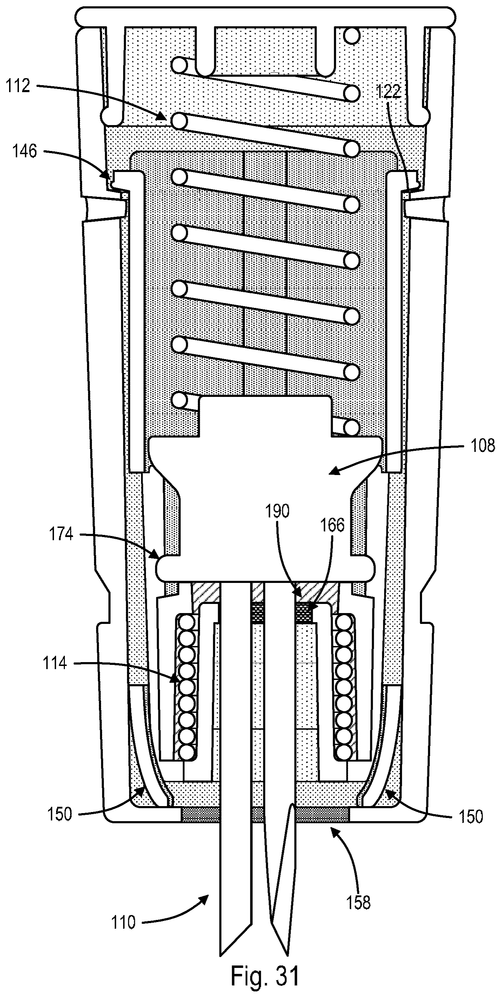

As previously discussed herein, the grooves **156** of the inner sleeve **106** and the projections **180** of the needle platform **108** guide the vertical movement of the needle platform **108.** As the needle platform moves in the direction of arrow **C,** the lower circular extension **174** contacts the deformable tabs **150 (****Fig. 31****).** This contact causes the tabs **150** to deflect away from the spring sleeve **182** of the lancet **100** or causes the tabs **150** to break. As depicted in **Fig. 32****,** when the tabs **150** break, the tabs **150** fall into a void between the inner sleeve **106** and the housing **102.** In the deployed position, the needles **110** extend beyond the housing **102** and the lower circular extension **174** contacts the top wall **186** of the spring sleeve **182.** Once the tabs 150 break, the lancet cannot be used again (i.e., single use). This is because once the retraction spring **114** is released, the blades are held back and are no longer set in the correct position.

When the tabs **150** deflect away from the spring sleeve **182** or break, the tabs **150** no longer contact the ledge **192** of the spring sleeve **182.** As a result, the retraction spring **114** is allowed to expand. The expansion of the retraction spring **114** moves the spring sleeve **182** vertically upward in the direction of arrow **D.** If the tabs **150** deflect and do not break, the spring sleeve **182** prevents the tabs **150** from contacting the retraction spring **114** as the retraction spring **114** expands. The spring sleeve **182** moves the needle platform **108** in the direction of arrow **D** to place the lancet **100** in the retracted position. That is, after moving to the deployed position, the retraction spring **114** causes the needles **110** to retract back into the inner volume **142** of the inner sleeve **106** the well opening **306** of the bottom layer **300,** the needle aperture **510** of the top layer **500,** the aperture **128** of the housing **102,** and the opening **158** of the inner sleeve **106.** That is, after penetrating the skin of a subject, the retraction spring **114** causes the needles **110** to automatically retract back into the housing of the lancet **100** thereby placing the lancet **100** in the retracted position.

By simultaneously moving three needles **110** to the deployed position, the lancet **100** punctures the skin of the subject at three locations at a same time. The three needles cause the tensioning of the skin such that one of the punctures created by the needles **110** penetrates the skin at a different depth. As a result, it is possible to have a higher volume of blood flow out of one of the punctures relative to the other two. This can occur when some of the needles tent slightly more such that the other needle(s) puncture a portion of the skin that is slightly more tensioned. That is, the three needles **110** are positioned on the needle platform **108** such that one of the needles **110** draws a majority of the physiological sample. Also, using three needles increases the chances of penetrating a capillary and puncturing the skin, thereby resulting in a more effective blood draw. The spring sleeve can help with the alignment of the needles. Also, the orientation of the needles can be selected to increase the distance between puncture points. For example, when the blade tips are pointed outward, the deepest points of puncture are further away from each other compared to when the blade tips are pointed inwards. Having a greater distance between puncture points can increase the likelihood that a capillary is targeted, thereby increasing the probability of bleeding. However, when the blade tips are pointed outward, less skin can be pinched, which can reduce the amount of bleeding. Overall, it has been found that there is better bleeding when the blade tips are pointed outward.

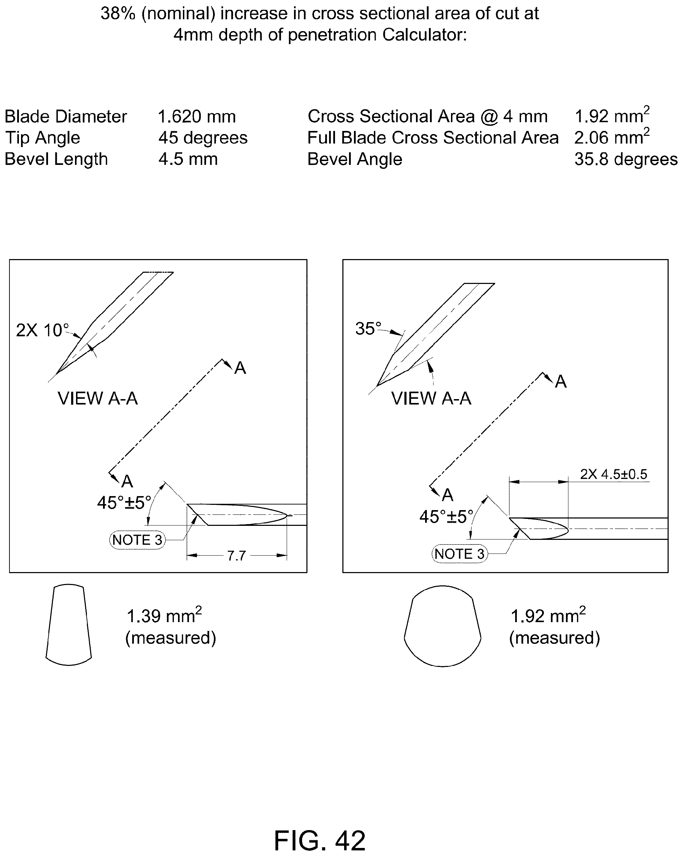

The needles 110 can be coated with silicone to minimize friction and tenting of the skin, which helps prevent the needles from sticking to the skin, thereby helping to minimize injection pain. Specifically, Silbione^{™} medical grade silicone fluids, such as Elkem and Nusil, can be used. This can also reduce break-loose force and insertion and drag forces. Also, the geometry of the blades of the needles 110 can be selected to promote bleeding. The blade tip geometry variables that impact the width and diameter of skin penetration include bevel length, bevel angle, tip angle and blade diameter. The blade diameter can be selected to be in the range of approximately 1.5 mm to 1.69 mm, preferably 1.60 to 1.64 mm, for example 1.62 mm, as shown in **FIG. 42****.** The tip angle can be selected to be in the range of approximately 15 degrees to 75 degrees, preferably 45 degrees +/= 5 degrees. The bevel length can be selected to be in the range of approximately 3 mm to 8 mm, for example 4.5 mm +/- 0.5 mm. Also, the bevel angle can be selected to be in the range of approximately 15 degrees to 75 degrees, for example 35 degrees. These blade dimensions can be selected to increase the cross sectional surface area of the blade 4 mm from the tip of the blade, as shown in **FIG. 42****,** which can create a wider wound and result in more bleeding.

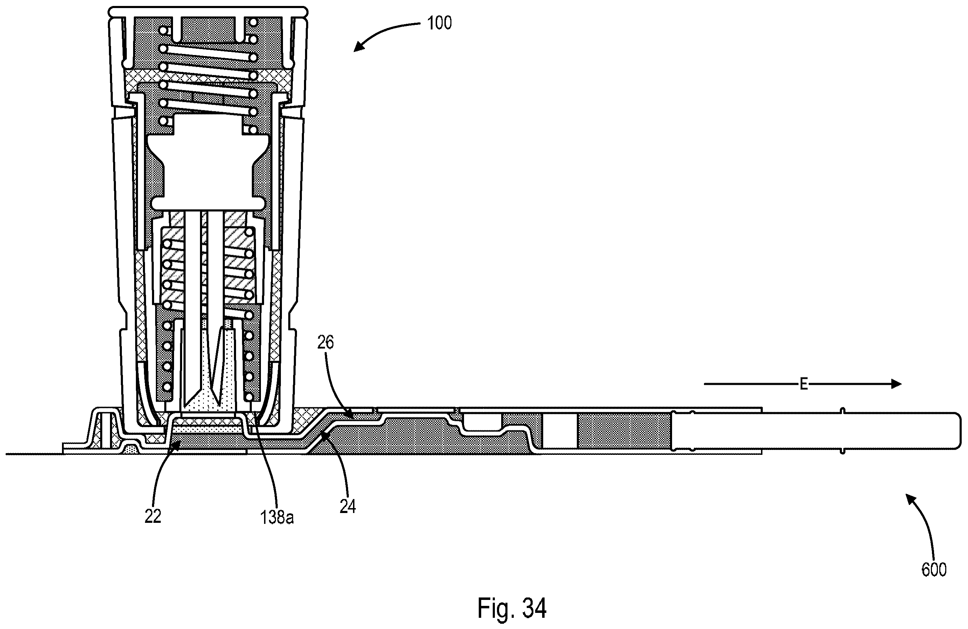

After the needles **110** retract, a physiological sample pools within the physiological sample well **22** of the cartridge **12.** When the physiological sample is within the physiological sample well **22,** a user can pull the vacuum pin **600** in the direction of arrow **E (****Fig. 34****)** to move the vacuum pin **600** from a first position (also referred to as an "undeployed" position) to a second position (also referred to as an "deployed" position). Moving the vacuum pin **600** to the deployed position creates a vacuum the cartridge **12.** More specifically, since the desiccant **400** is formed of a porous material, moving the vacuum pin **600** to the deployed position creates a vacuum within the first channel **24** and the second channel **26** via the grooves **324** of the collection pad support **310.** The strength of the vacuum is proportional to the amount the vacuum pin **600** moves. That is, the more the vacuum pin **600** moves, the stronger the vacuum. This vacuum causes the drawn physiological sample to travel to the collection pads **16** via the first channel **24** and the second channel **26.** In some embodiments, capillary flow, wicking and gravity assist the vacuum pin **600** in drawing the physiological sample towards the collection pads **16.** The collection pads **16** absorb the drawn physiological sample. The user can determine the collection pads **16** have absorbed the physiological sample by viewing the collection pads **16** through the viewing apertures **712** of the adhesive cover **700.** Furthermore, the hydrophobic foam **18** prevents physiological sample from exiting the collection pads **16** as the hydrophobic foam **18** repels physiological sample back towards the collection pads **16.** The desiccant material of the desiccant **400** then dries the physiological sample on the collection pads **16** via the side grooves **322** side grooves 322.

After the collection pads **16** have absorbed the physiological sample, the user can remove the cartridge 12 from the subject's skin. The user can then send the cartridge **12** to a laboratory where a medical professional can expose the collection pads **16** by removing the second portion **716** from the third portion **718** of the adhesive cover **700** as previously discussed herein. Once removed, the medical professional can apply various solutions to the collection pads **16** which mixes with the physiological sample to form a processed physiological sample that has been freed from the collection pads **16.** The processed physiological can then be analyzed.

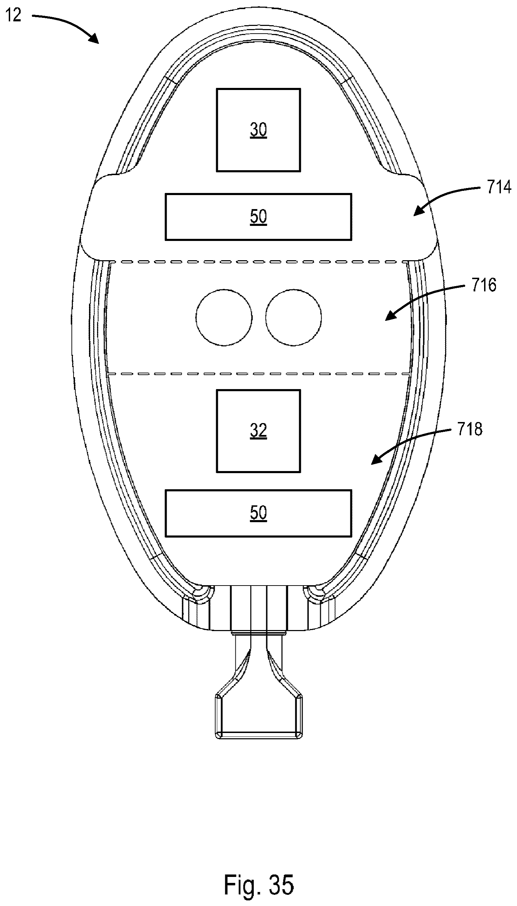

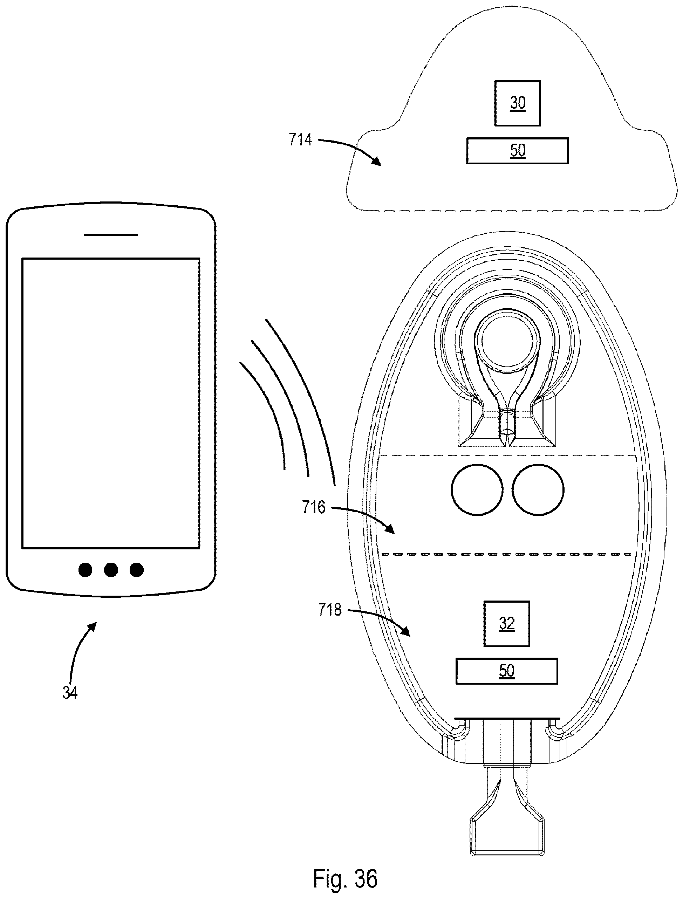

With reference to **Fig. 35** and **Fig. 36****,** in some embodiments, the adhesive cover **700** includes a first quick response ("QR") code **30** disposed on the first portion **714** of the adhesive cover **700** and a second QR code **32** disposed on the third portion **718** of the adhesive cover **700.** In one embodiment, the QR codes **30** and **32** can be used to establish a chain of custody of the cartridge **10.** For example, after drawing a physiological sample as previously discussed herein, a user or the subject may keep the first portion **714** of the adhesive cover **700** in their possession and can send the cartridge **12** with the drawn physiological sample to a laboratory for further analysis. In this embodiment, a computer system **34** that includes or is in communication with a QR code database **36** scans the QR codes **30** and **32.**

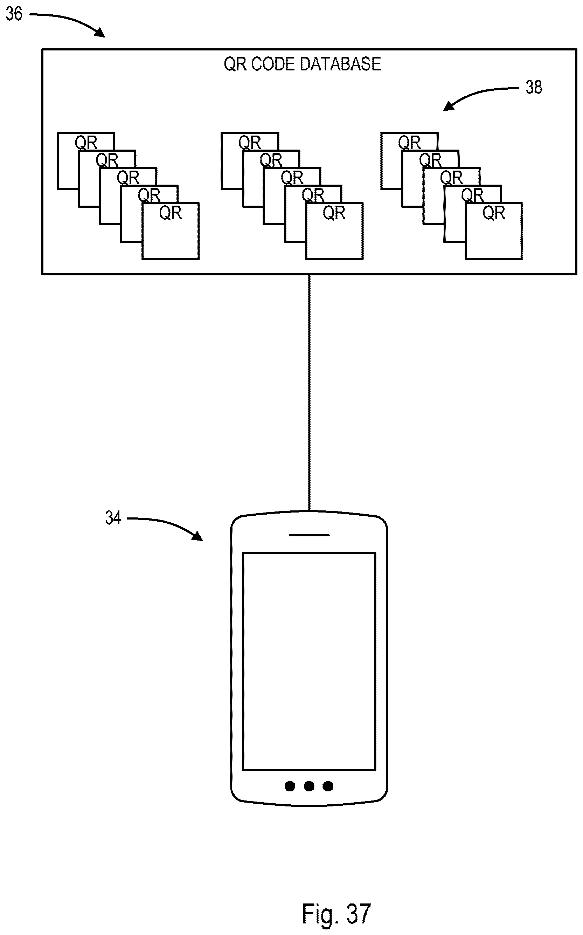

With reference to **Fig. 37****,** the QR code database **36** includes a plurality of QR codes **38** each of which are associated with a single cartridge **12.** In response to scanning the QR codes **30** and **32,** the computer system **34** determines if the QR codes are associated with a same cartridge **10** by comparing the scanned QR codes **30** and **32** to the QR codes **38** in the QR code database **36.** In response to determining that the QR codes **30** and **32** are associated with the same cartridge **10,** the computer system **34** determines that chain of custody has been preserved and outputs a notification indicating chain of custody has been preserved. In response to determining that the QR codes **30** and **32** are associated with different cartridges **10,** the computer system **34** determines that chain of custody has not been preserved and outputs a notification indicating chain of custody has not been preserved. In another embodiment, the QR codes **30** and **32** are the same. In this embodiment, the computer system **34** scans the QR codes **30** and **32** and determines that chain of custody has been preserved if the QR codes **30** and **32** are the same. In response to determining that the QR codes 30 and **32** are the same, the computer system **34** outputs a notification that chain of custody has been preserved. In response to determining the QR codes **30** and **32** are different, the computer system **34** outputs a notification that chain of custody has not been preserved.

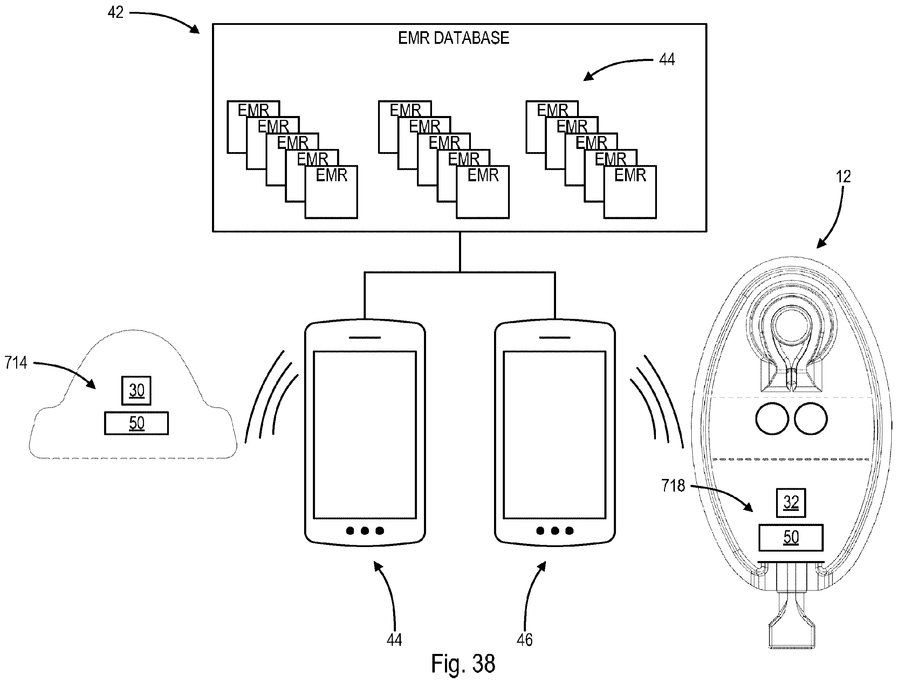

In another embodiment (Fig. 38), a first computer system 44 is connected to an electronic medical record ("EMR") database 42 that includes a plurality of EMRs 44 each associated with a different subject. In this embodiment, after drawing a physiological sample from a subject, the first computer system 44 scans the first QR code 30 and sends the cartridge 12 to a laboratory for further analysis. In response to scanning the first QR code 30, the first computer system 40 associates the first QR code 30 and the second QR code 32 with an EMR 44 that corresponds to the subject from which the physiological sample was drawn. In some embodiments, the first computer system 44 may update the associated EMR 44 to indicate that a physiological sample has been drawn automatically or based on a user input. A user of a second computer system 46 receives the cartridge 12 and scans the second QR code 32. The second computer system 46 determines an identity of the subject by matching the QR code **32** to the EMR **44** that is associated with the first and second QR codes **30** and **32.** While the above describes a system for identifying a subject from which a physiological sample has been drawn by matching QR codes in an EMR database, the system may identify a subject from which a physiological sample has been drawn by matching QR codes in any database that includes a subject's identity.

As depicted in **Fig. 35****,** the first portion **714** and the third portion **718** of the adhesive cover **700** can also include a serial number **50.** The serial number **50** can also be used to establish a chain of custody as previously discussed herein with respect to the QR codes **30** and **32.**

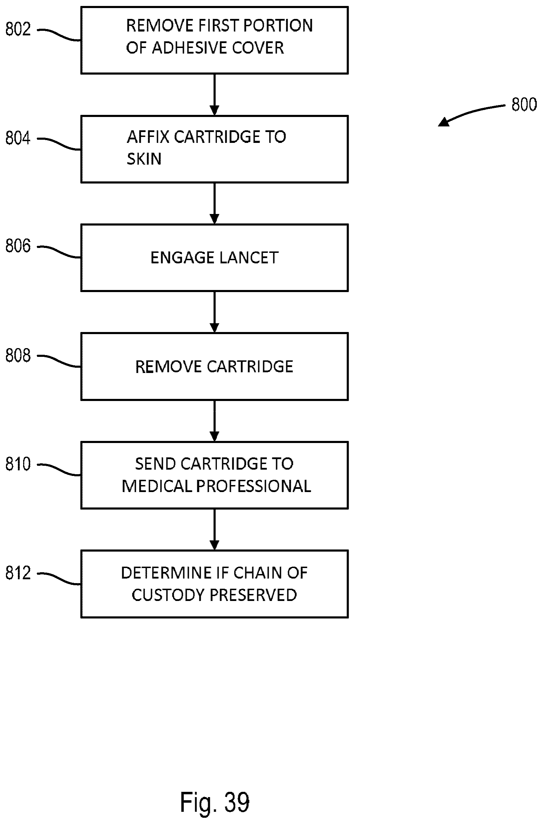

Referring now to **Fig. 39****,** a method **800** for obtaining a physiological sample from a subject is disclosed herein.

At **802,** a user or a subject removes the first portion **714** of the adhesive cover as previously discussed herein.

At **804** the cartridge **12** of the dermal patch system **10** is affixed to the skin of a subject as previously discussed herein.

**At 806,** the lancet **100** is engaged with the cartridge **12** to draw a physiological sample (e.g., a blood sample, a sample of interstitial fluid, etc.) from the subject as previously discussed herein.

**At 808,** the cartridge **12** is removed from the skin of the subject as previously discussed herein.

**At 810,** the cartridge **12** is sent to a medical professional to analyze the drawn physiological sample as previously discussed herein.

**At 812,** a computer system determines if chain of custody has been preserved and optionally updates an electronic medical record to indicate a physiological sample has been obtained from a subject as previously discussed herein.

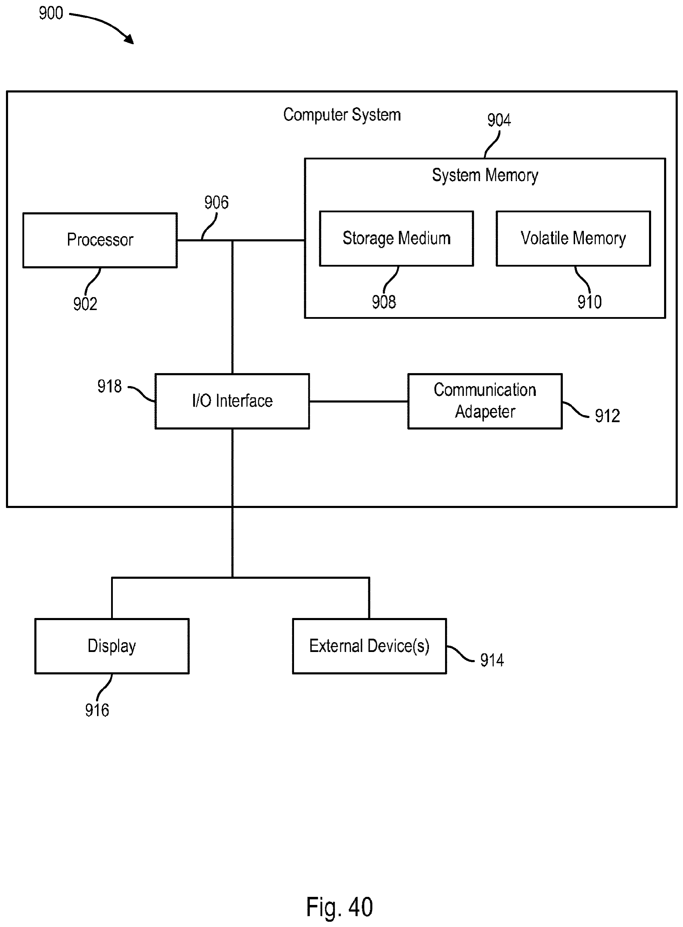

Referring now to **Fig. 40****, a** computer system **900** is shown in accordance with an exemplary embodiment. The computer system **900** may serve as any computer system disclosed herein (e.g., the computer system **40,** computer system **46).** As used herein a computer system (or device) is any system/device capable of receiving, processing, and/or sending data. Computer systems include, but are not limited to, microprocessor-based systems, personal computers, servers, hand-held computing devices, tablets, smartphones, multiprocessor-based systems, mainframe computer systems, virtual reality ("VR") headsets and the like.

As shown in **Fig. 40****, the** computer system **900** includes one or more processors or processing units **902,** a system memory **904,** and a bus **906** that couples the various components of the computer system 900 including the system memory **904** to the processor **902.** The system memory **904** includes a computer readable storage medium **908** and volatile memory **910** (e.g., Random Access Memory, cache, etc.). As used herein, a computer readable storage medium includes any media that is capable of storing computer readable; program instructions and is accessible by a processor. The computer readable storage medium **908** includes non-volatile and non-transitory storage media (e.g., flash memory, read only memory (ROM), hard disk drives, etc.). Computer program instructions as described herein include program modules (e.g., routines, programs, objects, components, logic, data structures, etc.) that are executable by a processor. Furthermore, computer readable program instructions, when executed by a processor, can direct a computer system to function in a particular manner such that a computer readable storage medium comprises an article of manufacture. Specifically, the computer readable program instructions when executed by a processor can create a means for carrying out at least a portion of the steps of the methods disclosed herein.

The bus **906** may be one or more of any type of bus structure capable of transmitting data between components of the computer system **900** (e.g., a memory bus, a memory controller, a peripheral bus, an accelerated graphics port, etc.).

The computer system **900** may further include a communication adapter **912** which allows the computer system **900** to communicate with one or more other computer systems/devices via one or more communication protocols (e.g., Wi-Fi, BTLE, etc.) and in some embodiments may allow the computer system **900** to communicate with one or more other computer systems/devices over one or more networks (e.g., a local area network (LAN), a wide area network (WAN), a public network (the Internet), etc.).

In some embodiments, the computer system **900** may be connected to one or more external devices **914** and a display **916.** As used herein, an external device includes any device that allows a user to interact with a computer system (e.g., mouse, keyboard, touch screen, etc.). An external device **914** and the display **916** may be in communication with the processor **902** and the system memory **904** via an Input/Output (I/O) interface **918.**

The display **916** may display a graphical user interface (GUI) that may include a plurality of selectable icons and/or editable fields. A user may use an external device **914** (e.g., a mouse) to select one or more icons and/or edit one or more editable fields. Selecting an icon and/or editing a field may cause the processor **902** to execute computer readable program instructions stored in the computer readable storage medium **908.** In one example, a user may use an external device **914** to interact with the computer system **900** and cause the processor **902** to execute computer readable program instructions relating to at least a portion of the steps of the methods disclosed herein.

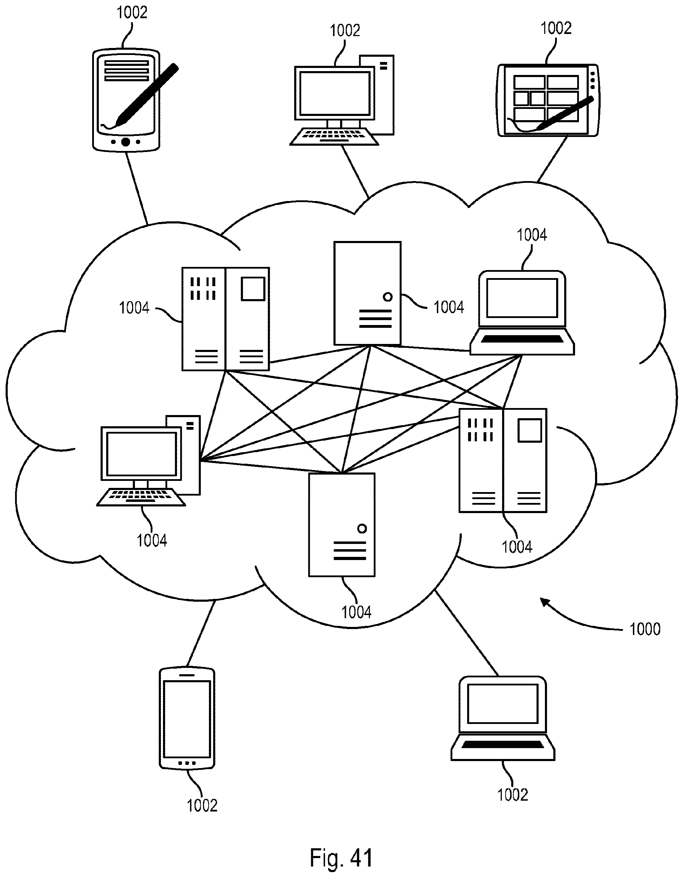

Referring now to **Fig. 41****,** a cloud computing environment **1000** is depicted in accordance with an exemplary embodiment. The cloud computing environment **1000** is connected to one or more user computer systems **1002** and provides access to shared computer resources (e.g., storage, memory, applications, virtual machines, etc.) to the user computer systems **1002.** As depicted in **Fig. 40****,** the cloud computing environment includes one or more interconnected nodes **1004.** Each node **1004** may be a computer system or device local processing and storage capabilities. The nodes **1004** may be grouped and in communication with one another via one or more networks. This allows the cloud computing environment **1000** to offer software services to the one or more computer services to the one or more user computer systems **1002** and as such, a user computer system **1002** does not need to maintain resources locally.

In one embodiment, a node **1004** includes computer readable program instructions for carrying out various steps of various methods disclosed herein. In these embodiments, a user of a user computer system **1002** that is connected to the cloud computing environment may cause a node **1004** to execute the computer readable program instructions to carry out various steps of various methods disclosed herein.

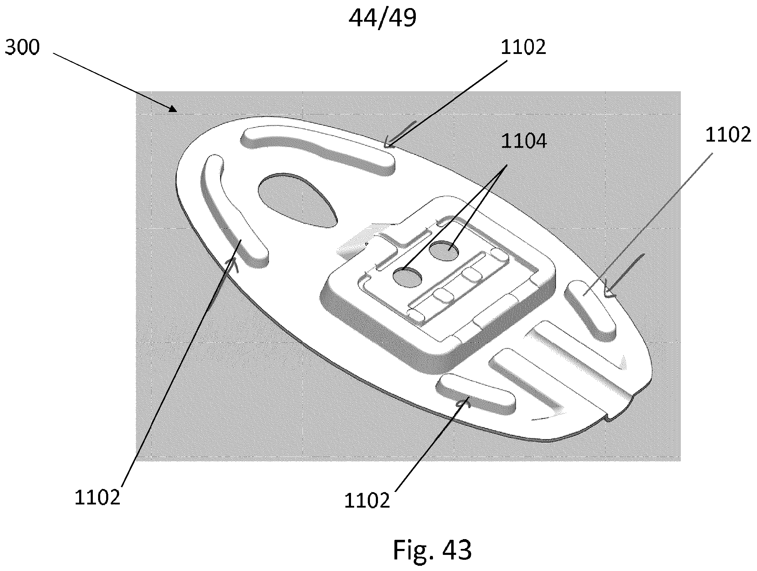

**Fig. 43** depicts a bottom layer **300** of the cartridge **12** in accordance with another embodiment of the present disclosure. In this embodiment, the cartridge **12** includes alignment ridges **1102** that can be used to align the bottom layer **300** with the top layer **500.** The bottom layer **300** also can include cutout vent holes **1104** that can be used to allow the collected sample on the collection pads **16** to dry faster after collection of the sample. One or more vent holes can be used. These cutout vent holes can also be made in the bottom adhesive layer **200,** but not necessarily the liner **14.**

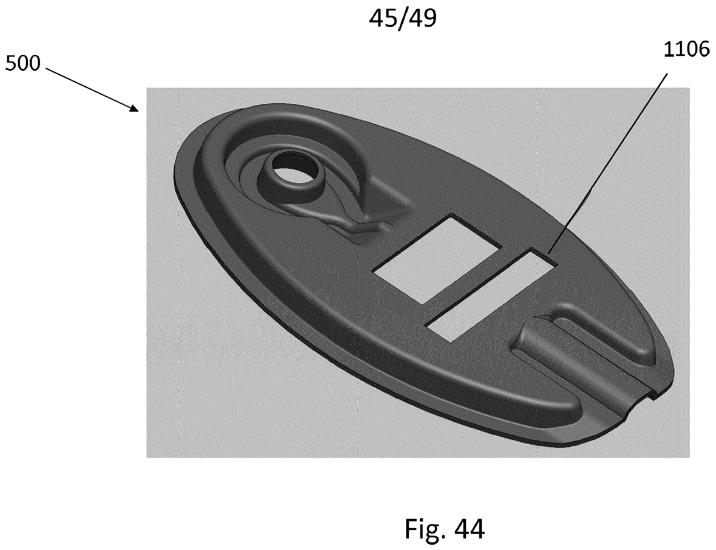

**Fig. 44** depicts a top layer **500** of the cartridge **12** in accordance with another embodiment of the present disclosure. The top layer **500** includes cutout **1106** for an integrated overflow filter material that can improve the ease of manufacturing.

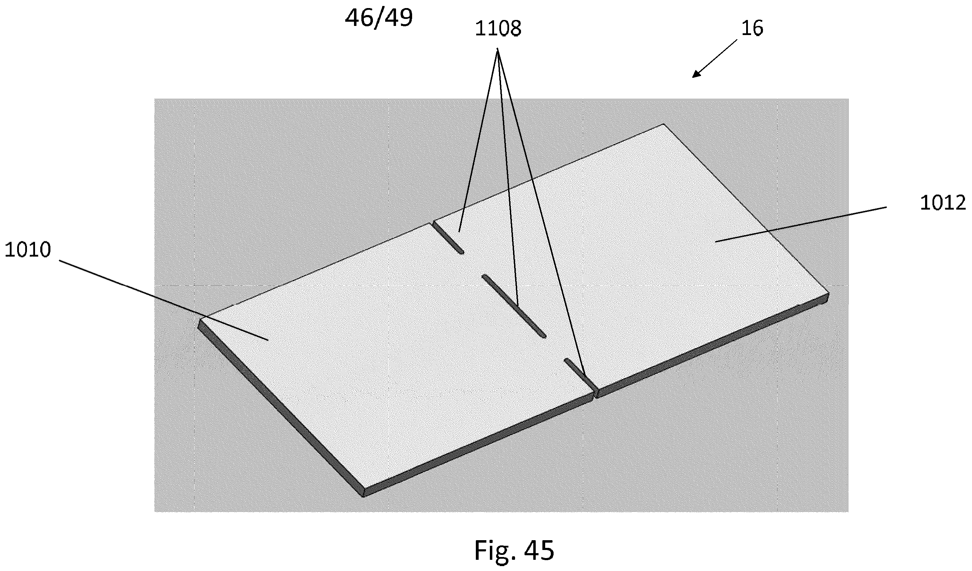

**Fig. 45** depicts a collection pad **16** in accordance with another embodiment of the present. The collection pad **16** can be made as one piece with tabs **1108** formed at a middle point therein, thereby forming a first section **1010** and a second section **1012.** The tabs **1108** can be formed as perforations die cut into the pad. The first section **1010** and second section **1012** of the collection pad 16 can be separated by pulling the sections apart and breaking the tabs. The two (2) pieces of the collection pad 16 can be approximately 8 mm x 8mm. Because the collection pad 16 of this embodiment can be made as one piece, as opposed to two (2) pieces that require alignment, it is easier to manufacture the collection pad. However, it is not necessary to manufacture the collection pad 16 as one piece.

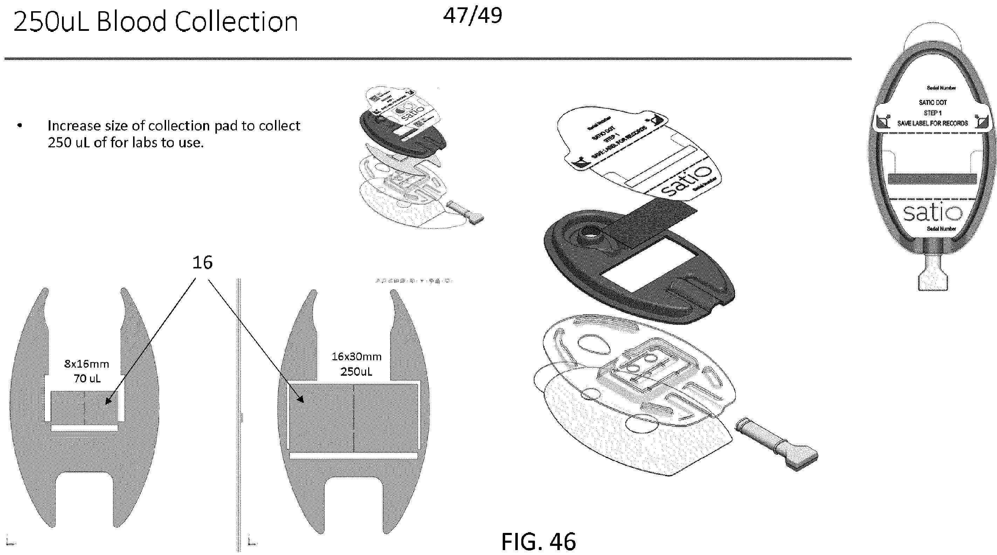

The size of the collection pad can be selected to collect a larger physiological sample. For example, the collection pad can be increased from a 70µL capacity (approximately 8 mm x 16 mm) to 250µL capacity (approximately 16 mm x 30 mm), as shown in FIG. 46. The size of the pad can be increased to provide a larger collection capacity. This increased volume can be useful in order to separate blood plasma from the blood sample, as presented below. An overflow area can be used in the vicinity of the pad to limit the saturation of blood on the pad in order to keep the collected blood volume within a targeted range of blood volume. As shown in FIG. 46, added material in the periphery of the rectangular pad can be used to draw excess blood from the collection well, thereby preventing over saturation of the rectangular pad, ensuring accuracy of the collected volume as well as assisting with the drying of the sample on the pad for sample viability.

The collection pad can be configured to separate a plasma sample from the physiological/blood sample. A blood sample of approximately 20 - 250 µL can be used to obtain a plasma sample of approximately 5 - 200 µL. This can be done by including a plasma filter in the collection pad that includes a separator that separates the plasma from the blood. For example, a Whatman Fusion 5 pad can be used in place of the collection pad 16. As described above with reference to collection pad 16 in FIGS. 13 and 25, viewing apertures can be aligned with the Fusion 5 pad, which can be covered with a transparent film that allows a user of the dermal patch system to view the Fusion pad in order to visually confirm blood plasma collection. Also, the cover can include a transparent portion that allows a user to view the plasma collection pad. Similar to the collection pad 16 described above, the Fusion 5 pad can be sent to a lab for analysis of the plasma. Upon receipt of the sample, a section containing the separated plasma can be torn away.

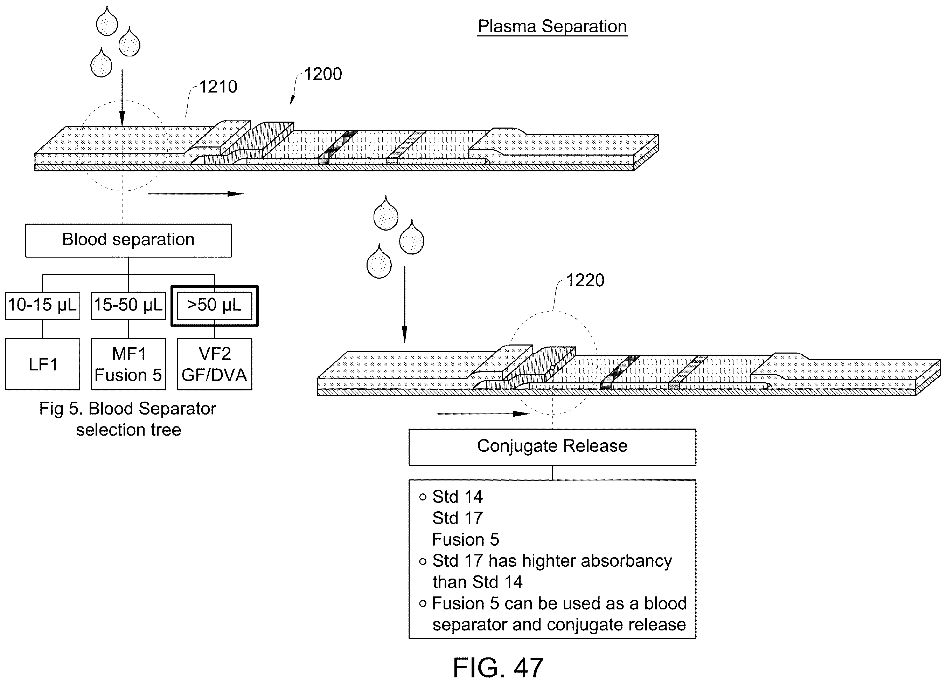

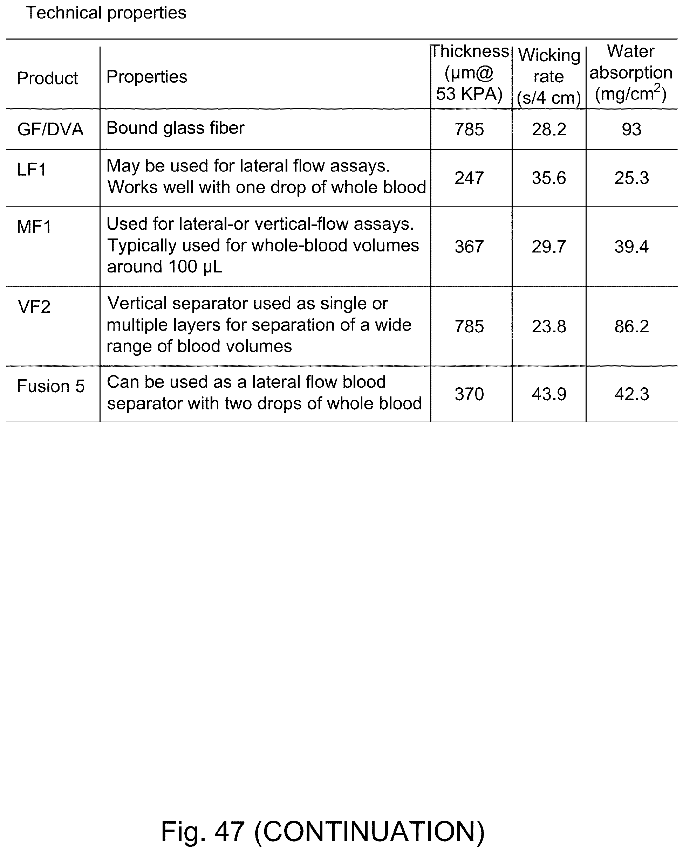

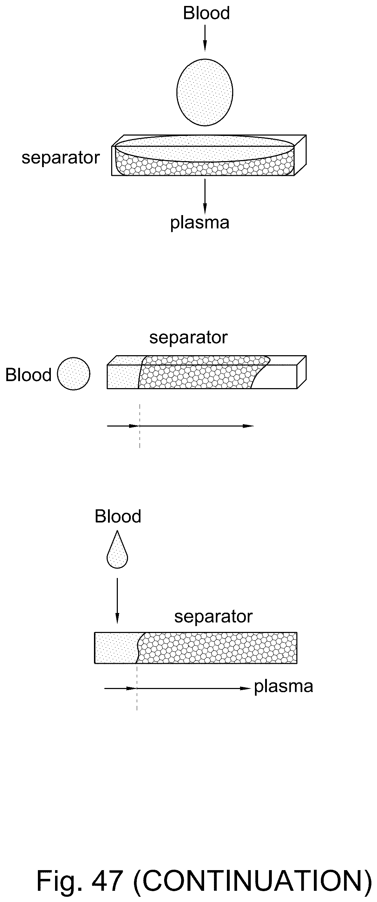

An example plasma filter **1200** is shown in FIG. 47. The filter **1200** includes a blood separation portion **1210** and a conjugate release portion 1220. In the blood separation portion **1210,** a vertical flow separator or a lateral flow separator can be used to separate the plasma from the blood. For example, an LF1 separator can be used for lateral flow assays, and works well with one drop of blood (approximately 10-15 µL). Also, an MF1 separator can be used for lateral or vertical flow assays for a blood volume of about 100 µL, or 15-50 µL. A VF2 vertical separator can be used as a single or multiple layers for separation of a wide range of blood volumes (e.g., >50 µL). A Fusion 5 separator can be used as a lateral flow blood separator using two drops of blood (approximately 15-50 µL), and can be used for conjugate release. A Whatman GF/DVA bound glass separator can be used for separation of a wide range of blood volumes (e.g., >50 µL). A Fusion 5 separator also can be used for conjugate release in the conjugate release portion 1220. Alternatively, an Std 17 or Std 14 can be used for the conjugate release. A Std 17 conjugate release has a higher absorbency than a Std 14 conjugate release. Also, GF, GX or GR Vivid Plasma Separation Membranes, a Cytosep Membrane Media Grade 1660 or an MDI Membrane Technologies FR1 (IPD) can be used for plasma separation.

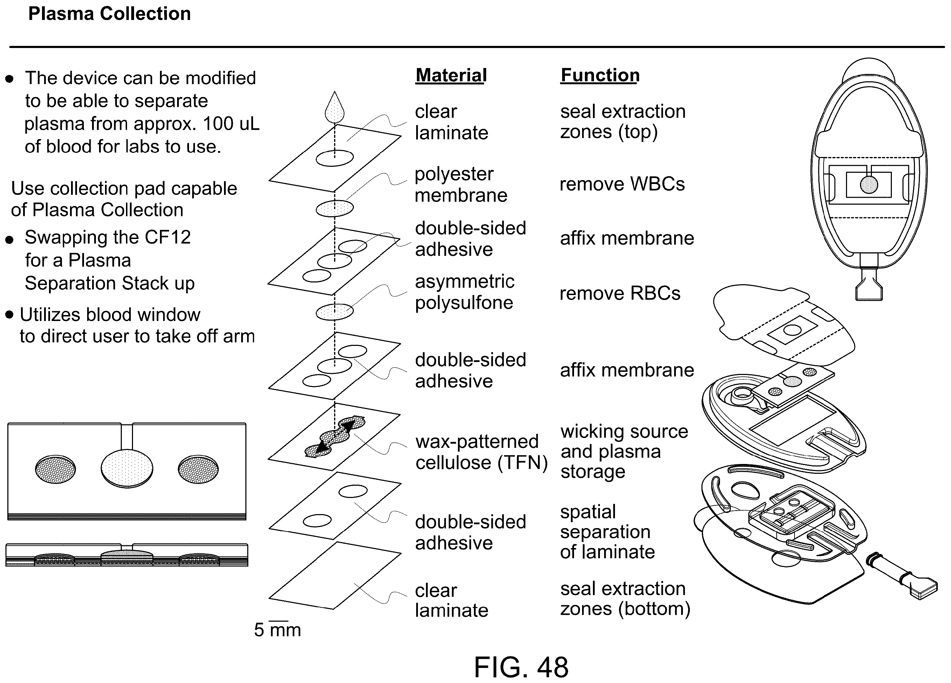

**FIG. 48** shows a sample collection pad that can be used to collect plasma. As shown in FIG. 48, the plasma collection pad can include several layers including top and bottom clear laminate layers that can be used to seal the top and bottom extraction zones, respectively. Below the top clear laminate layer is a polyester membrane that can be used to remove white blood cells (WBC's). Below the polyester membrane is a first double-sided adhesive that adheres to the polyester membrane. Below the first double-sided adhesive is an asymmetric polysulfone layer that is adhered thereto and can be used to remove red blood cells (RBC's). Below the asymmetric polysulfone layer is a second double-sided adhesive that adheres to the asymmetric polysulfone layer. Below the second double-sided adhesive is a wax-patterned cellulose (TFN) layer that provides a wicking source and plasma storage. Below the wax-patterned cellulose (TFN) layer is a third double-sided adhesive that adheres the wax-pattemed cellulose (TFN) layer to the bottom clear laminate layer. When a blood sample is applied to the plasma collection, plasma will be separated from the blood sample and stored in the wax-patterned cellulose (TFN) layer. The plasma collection pad can be covered with a transparent film that allows a user of the dermal patch system to view the collection pad in order to visually confirm blood plasma collection through viewing apertures. Also, the cover can include a transparent portion that allows a user to view the plasma collection pad.

As previously discussed, the above may be implemented by way of computer readable instructions, encoded or embedded on computer readable storage medium (which excludes transitory medium), which, when executed by a processor(s), cause the processor(s) to carry out the methods of the present disclosure.

In some embodiments, a method for drawing a physiological sample from a subject includes the steps of: affixing a cartridge of a dermal patch system to the skin of a subject, engaging a lancet with the cartridge to draw a physiological sample, removing the cartridge from the skin of the subject, and determining if chain of custody of the cartridge has been preserved by scanning a quick response code of the cartridge. The method can also include a step of using a lancet that may include one or more needles that are configured to move from an undeployed position to a deployed position upon engagement with the cartridge. The lancet may be configured to automatically retract the one or more needles. The method can also include a step of pulling a vacuum pin of the cartridge to draw the physiological sample to a sample collection pad of the cartridge.

In some embodiments, a system for collecting a physiological sample from a subject includes: a lancet configured to draw a physiological sample, and a cartridge configured to receive and store the physiological sample. The cartridge can include: a cover with a first portion and a second portion, wherein the first portion is removable, a first quick response code disposed on the first portion of the cover; and a second quick response code disposed on the second portion, wherein the first and second quick response codes are associated with the cartridge. The cartridge can further include a sample collection pad, wherein the cover seals the sample collection pad within the cartridge. The cover can include a transparent portion that allows a user to view the sample collection pad. The cover can further include a third portion that covers the sample collection pad and includes the transparent portion, wherein the third portion is removable. The lancet can include one or more needles that are moveable between an undeployed position and a deployed position, wherein the one or more needles are configured to draw the physiological sample when in the deployed position. The cartridge can further include a lancet receiving element configured to cause the lancet to move the one or more needles to the deployed position, wherein the first portion covers the lancet receiving element. The first portion can include a pull tab which allows a user to remove the first portion. The cartridge can include a moveable vacuum pin configured to create a vacuum within the cartridge when moved, wherein the vacuum draws the physiological sample to the sample collection pad.

While various embodiments have been illustrated and described in detail in the drawings and foregoing description, such illustration and description are to be considered illustrative or exemplary and not restrictive; embodiments of the present disclosure are not limited to the disclosed embodiments. Other variations to the disclosed embodiments can be understood and effected by those skilled in the art in practicing embodiments of the present disclosure, from a study of the drawings, the disclosure, and the appended claims.

In the claims, the word "comprising" does not exclude other elements or steps, and the indefinite article "a" or "an" does not exclude a plurality. A single processor or other processing unit may fulfill the functions of several items recited in the claims. The mere fact that certain measures are recited in mutually different dependent claims does not indicate that a combination of these measured cannot be used to advantage. Any reference signs in the claims should not be construed as limiting the scope.

## Claims

1. A system for collecting a physiological sample from a subject comprising:
a lancet configured to draw a physiological sample, and
a cartridge configured to be affixed to the subject's skin and including:
a physiological sample well,
a sloped physiological sample channel in open communication with the physiological sample well,
a sample collection pad in open communication with the sloped physiological channel and configured to absorb the drawn physiological sample,
wherein the physiological sample well is configured to retain the drawn physiological sample and the sloped physiological channel is configured to carry the drawn physiological sample from the physiological sample to the sample collection pad.

2. The system of claim 1, further comprising:
a desiccant disposed within the cartridge; and
a hydrophobic foam disposed within the cartridge and configured to prevent the drawn physiological sample from escaping the sample collection pad.

3. The system of claim 1, wherein the sample collection pad is a first sample collection pad and the cartridge includes a second collection pad,
wherein the physiological sample channel is configured to carry the drawn physiological sample to the first and second collection pad, and
wherein, optionally, the system further comprises an overflow area in the vicinity of the pad configured to limit the saturation of blood on the pad in order to keep the collected blood volume within a targeted range.

4. The system of claim 1, wherein the lancet includes:
one or more needles configured to move between an undeployed position and a deployed position, wherein the one or more needles are disposed within the lancet when in the undeployed position and extends out of the lancet when in the deployed position to draw the physiological sample.

5. The system of claim 4, wherein the cartridge includes:
a lancet receiving element,
wherein the lancet is configured to move the one or more needles from the undeployed position to the deployed position when the lancet engages with the lancet receiving element, and wherein, optionally, the lancet is configured to automatically retract the one or more needles into a housing of the lancet.

6. The system of claim 1, further comprising:
a vacuum pin disposed within the cartridge and configured to move between a deployed position and an undeployed position,
wherein the vacuum pin is configured to create a vacuum within the cartridge when moved from the undeployed position to the deployed position and the vacuum draws the drawn physiological sample from the physiological sample well to the sample collection pad,
wherein the cartridge includes:
a first layer of material, and
a second layer of material, and
wherein the first and second layer of material define the physiological sample channel.

7. The system of claim 1, wherein sample collection pad comprises sections with tabs therebetween.

8. The system of claim 1, wherein the cartridge comprises a bottom layer, an adhesive layer attached to the bottom layer, a top layer, a sample well opening formed in the adhesive layer, a needle aperture formed in the top layer, wherein the needle aperture is smaller than the sample well opening, and wherein the adhesive layer is formed of a pressure sensitive adhesive; and further comprising a desiccant formed in a space between the top layer and the bottom layer.

9. The system of claim 4, wherein the lancet is configured to move the one or more needles from the undeployed position to the deployed position only when a configuration of the lancet engages with a matching configuration of the cartridge.

10. The system of claim 4, wherein the one or more needles are coated with silicone.

11. The system of claim 8, wherein the cartridge includes alignment ridges configured to align the bottom layer with the top layer.

12. The system of claim 8, wherein either one or both of the bottom layer and the adhesive layer includes at least one cutout vent hole configured to allow the physiological sample on the sample collection pad to dry faster, and
wherein the top layer includes a cutout configured to hold an integrated overflow filter.

13. The system of claim 1, wherein the sample collection pad is made from one piece having tabs formed at a middle portion thereof, thereby forming a first section and a second section that are configured to be separated from each other when the first and second section are pulled apart,
wherein, optionally, the first section and the second section each are approximately 8mm by 8mm, and
wherein, optionally, the first section and the second section each are approximately 16mm by 15mm.

14. The system of claim 1, wherein the sample collection pad is configured to separate a plasma sample from the physiological sample,
wherein, optionally, the sample collection pad comprises a blood separation section and a conjugate release section,
wherein, optionally, the blood separation section comprises a separator selected from a group consisting of an LF1 separator, an MF1 separator, a VF2 vertical separator, a Fusion 5 separator, and GF/DVA bound glass separator, and
wherein, optionally, the conjugate release section comprises a conjugate release element selected from a group consisting of a Std14, Std 17 and Fusion 5.

15. The system of claim 14, wherein the sample collection pad comprises a plasma separation stack up, wherein the plasma separation stack up comprises a membrane configured to remove white blood cells, a membrane configured to remove red blood cells and a layer configured to provide a wicking source and plasma storage, and wherein the plasma separation stack up comprises a top clear laminate layer configured to allow viewing of the plasma sample, a polyester membrane located below the top clear laminate layer and configured to remove white blood cells, a first double-sided adhesive located below the polyester membrane and configured to adhere to the polyester membrane, an asymmetric polysulfone layer located below the first double-sided adhesive and configured to remove red blood cells, a second double-sided adhesive located below the asymmetric polysulfone layer and configured to adhere to the asymmetric polysulfone layer, a wax-patterned cellulose layer located below the second double-sided adhesive and configured to provide a wicking source and plasma storage, a third double-sided adhesive located below the wax-patterned cellulose layer and configured to adhere to the wax-pattemed cellulose layer, and a clear laminate bottom layer located below the third double-sided adhesive.
